(19) Europäisches Patentamt
European Patent Office
Office européen des brevets

(11) Publication number: **0 427 680 B1**

# (12) EUROPEAN PATENT SPECIFICATION

(45) Date of publication of patent specification: **23.08.95**

(51) Int. Cl.[6]: **C07D 498/18**, A61K 31/33, //(C07D498/18,311:00,273:00, 221:00)

(21) Application number: **90810854.1**

(22) Date of filing: **07.11.90**

The file contains technical information submitted after the application was filed and not included in this specification

## (54) Heteroatoms-containing tricyclic compounds.

(30) Priority: **09.11.89 DE 3937336**
**16.11.89 DE 3938132**
**23.12.89 DE 3942831**
**23.12.89 DE 3942833**
**05.03.90 DE 4006819**

(43) Date of publication of application:
**15.05.91 Bulletin 91/20**

(45) Publication of the grant of the patent:
**23.08.95 Bulletin 95/34**

(84) Designated Contracting States:
**AT BE CH DE DK ES FR GB GR IT LI LU NL SE**

(56) References cited:
**EP-A- 0 184 162**
**EP-A- 0 227 355**
**EP-A- 0 323 042**

**JOURNAL OF THE AMERICAN CHEMICAL SOCIETY, vol. 109, 1987 H. TANAKA et al. "Structure of FK 506:"A novel immunosupressant isolated from Streptomyces"" pages 5031-5033**

(73) Proprietor: **SANDOZ LTD.**
**Lichtstrasse 35**
**CH-4002 Basel (CH)**
(84) Designated Contracting States:
**BE CH DK ES FR GB GR IT LI LU NL SE**

(73) Proprietor: **SANDOZ-PATENT-GMBH**
**Humboldtstrasse 3**
**D-79539 Lörrach (DE)**
(84) Designated Contracting States:
**DE**

(73) Proprietor: **SANDOZ-ERFINDUNGEN Verwaltungsgesellschaft m.b.H.**
**Brunner Strasse 59**
**A-1235 Wien (AT)**
(84) Designated Contracting States:
**AT**

(72) Inventor: **Baumann, Karl**
**Simmeringer Hauptstrasse 36/2/29**
**A-1110 Vienna (AT)**
Inventor: **Emmer, Gerhard**
**Theresiengasse 25**
**A-1180 Vienna (AT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 427 680 B1

## Description

The invention relates to the field of macrolides. It concerns the compounds of formula I

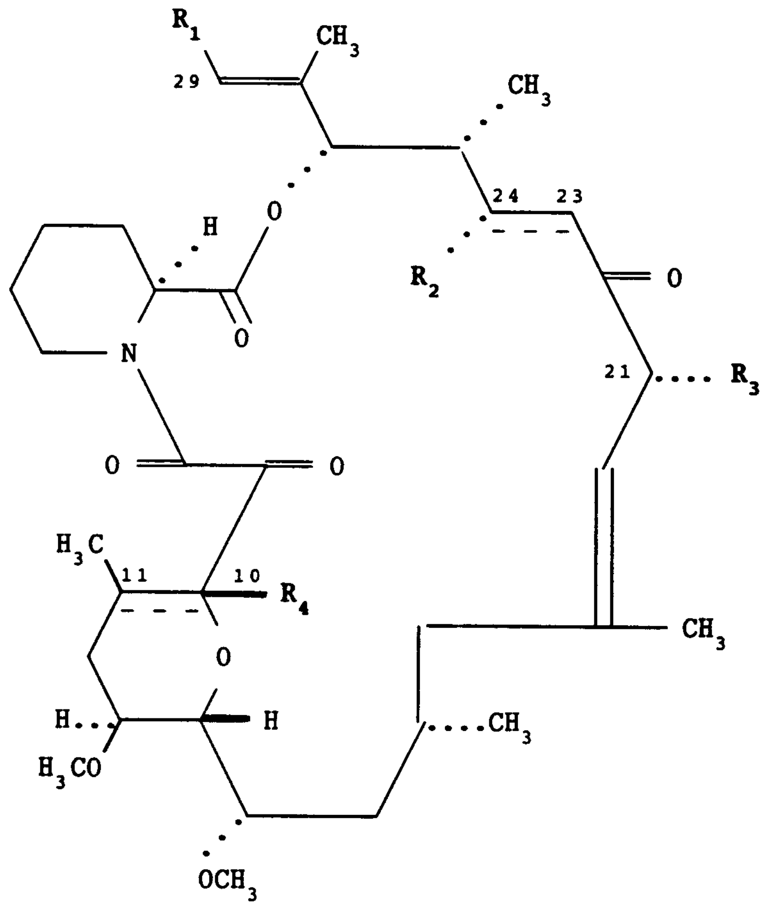

I

wherein
either
$R_1$ is a group (a) of formula

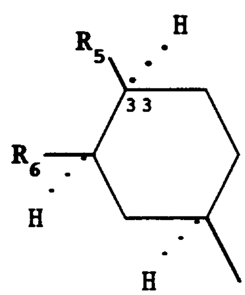

(a)

wherein
$R_5$ is chloro, bromo, iodo or azido and
$R_6$ is hydroxy or methoxy;
$R_2$ is oxo and there is a single bond in 23,24 position; optionally protected hydroxy and there is a single or a double bond in 23,24 position; or absent and there is a double bond in 23,24 position; and
$R_4$ is hydroxy and there is a single bond in 10,11 position; or absent and there is a double bond in 10,11 position;

or

$R_1$ is a group (b) or (d) of formula

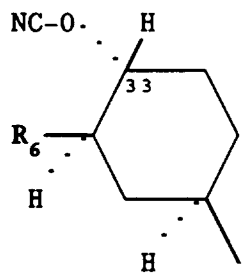 (b) and 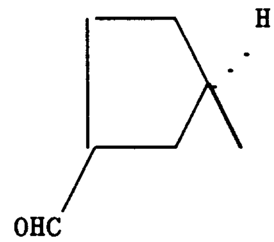 (d)

wherein

$R_6$ is as defined above;

$R_2$ is as defined above; and

$R_4$ is hydroxy and there is a single bond in 10,11 position; and

$R_3$ is methyl, ethyl, n-propyl or allyl;

in free form and, where such forms exist, in salt form, hereinafter referred to as "the compounds of the invention".

As is evident from formula I and the definition of the substituents when there is a single bond in 10,11 position the carbon atom to which the methyl group in 11 position is attached has the $\beta$-configuration and there is a hydrogen atom with the $\alpha$-configuration attached to the carbon atom in 11 position; when there is a double bond in 10,11 position this methyl group lies in the plane of the paper and there is no hydrogen atom in 11 position. When $R_2$ is oxo no hydrogen atom is attached to the carbon atom in 24 position.

$R_1$ preferably is a group (d). $R_2$ preferably is unprotected hydroxy and there is a single bond in 23,24 position. $R_3$ preferably is ethyl or allyl. $R_4$ preferably is hydroxy. $R_5$ preferably is chloro. $R_6$ preferably is methoxy.

Protected hydroxy preferably is hydroxy protected by a conventional hydroxy-protecting group such as formyl, tert-butoxycarbonyl, or trialkylsilyl; it especially is tert-butyldimethylsilyloxy.

A compound of the invention preferably is in free form. It preferably is in unprotected form.

A subgroup of compounds of the invention is the **compounds Ip$_1$**, i.e. the compounds of formula I wherein

$R_1$ is a group (a) wherein $R_6$ is methoxy and

either

$R_5$ is chloro or bromo and

$R_4$ is hydroxy and there is a single bond in 10,11 position

or

$R_5$ is azido and

$R_4$ is hydroxy and there is a single bond in 10,11 position or absent and there is a double bond in 10,11 position;

$R_2$ is optionally protected hydroxy and there is a single or a double bond in 23,24 position; and

$R_3$ is as defined above under formula I;

in free form and, where such forms exist, in salt form.

A further subgroup of compounds of the invention is the **compounds Ip$_3$**, i.e. the compounds of formula I wherein

$R_1$ is a group (b) wherein $R_6$ is methoxy,

$R_2$ is optionally protected hydroxy and there is a single bond in 23,24 position; or absent and there is a double bond in 23,24 position;

$R_4$ is hydroxy and there is a single bond in 10,11 position; and

$R_3$ is as defined above under formula I;

in free form and, where such forms exist, in salt form.

A further subgroup of compounds of the invention is the **compounds Ip$_4$**, i.e. the compounds of formula I wherein

$R_1$ is a group (d),

$R_2$ is optionally protected hydroxy and there is a single bond in 23,24 position; or absent and there is a double bond in 23,24 position;

$R_4$ is hydroxy and there is a single bond in 10,11 position; and

$R_3$ is as defined above under formula I;

in free form and, where such forms exist, in salt form.

A preferred subgroup of compounds of the invention is the compounds of formula I wherein

$R_1$ is a group (a) wherein $R_5$ is as defined above under formula I and $R_6$ is methoxy;

$R_2$ is optionally protected hydroxy and there is a single bond in 23,24 position;

$R_4$ is hydroxy and there is a single bond in 23,24 position; or absent and there is a double bond in 10,11 position; and

$R_3$ is ethyl or allyl.

A further preferred group of compounds of the invention is the compounds of formula I wherein

$R_1$ is a group (b) wherein $R_6$ is methoxy;

$R_2$ is optionally protected hydroxy and there is a single bond in 23,24 position; or absent and there is a double bond in 23,24 position;

$R_4$ is hydroxy and there is a single bond in 10,11 position; and

$R_3$ is ethyl or allyl.

A further preferred subgroup of compounds of the invention is the compounds of formula I wherein

$R_1$ is a group (d),

$R_2$ is optionally protected hydroxy and there is a single bond in 23,24 position; or absent and there is a double bond in 23,24 position;

$R_4$ is hydroxy and there is a single bond in 10,11 position; and

$R_3$ is ethyl or allyl.

A further subgroup of compounds of the invention is the **compounds Iq**, i.e. the compounds of formula I wherein

either

$R_1$ is a group (a) wherein $R_5$ is chloro, bromo, iodo or azido and

$R_6$ is hydroxy or methoxy,

$R_2$ is oxo and there is a single bond in 23,24 position; optionally protected hydroxy and there is a single or a double bond in 23,24 position; or absent and there is a double bond in 23,24 position; and

$R_4$ is hydroxy and there is a single bond in 10,11 position; or absent and there is a double bond in 10,11 position;

or

$R_1$ is a group (b) or (d) wherein $R_6$ is hydroxy or methoxy;

$R_2$ is as defined above for this subgroup; and

$R_4$ is hydroxy and there is a single bond in 10,11 position; and

$R_3$ is methyl, ethyl, n-propyl or allyl,

in free form and, where such forms exist, in salt form.

A further subgroup of compounds of the invention is the **compounds Ir**, i.e. the compounds of formula I wherein

either

$R_1$ is a group (a) as defined above under formula I; and

$R_2$ and $R_4$ have the significance indicated above under group (a);

or

$R_1$ is a group (b) or (d) as defined above under formula I; and

$R_2$ and $R_4$ have the significance indicated above under groups (b) and (d); and

$R_3$ is as defined above under formula I;

in free form and, where such forms exist, in salt form.

In a subgroup of compounds Ir $R_2$ is other than absent.

A further subgroup of compounds of the invention is the **compounds of formula Is**

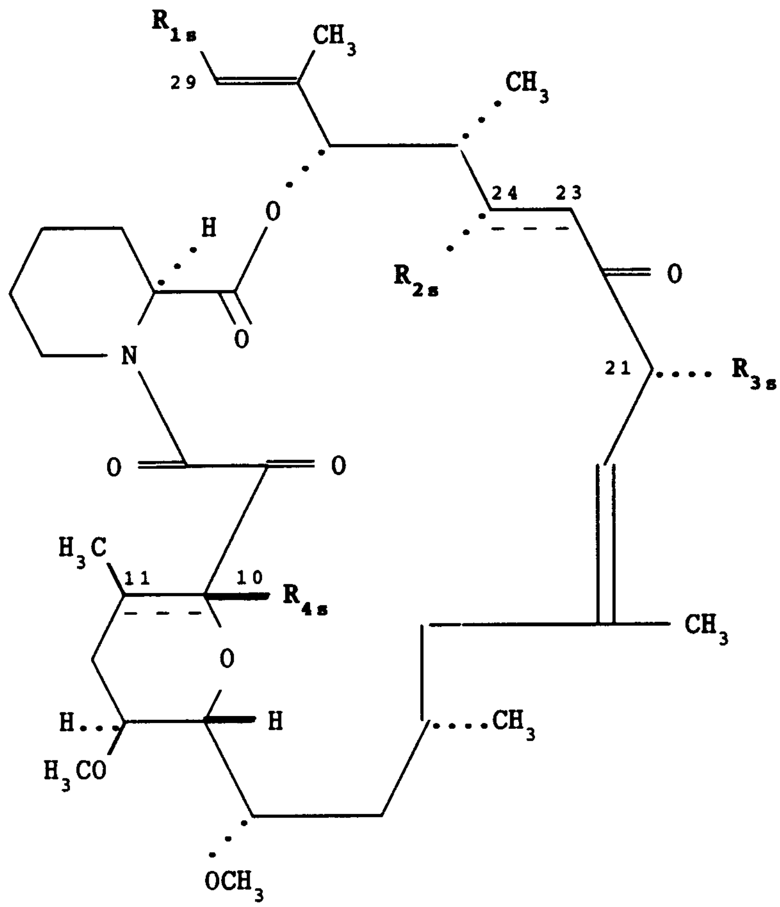

Is

wherein
either

$R_{1s}$ is a group (a) wherein $R_5$ is chloro, bromo, iodo or azido and $R_6$ is methoxy;
$R_{2s}$ is hydroxy optionally protected by tert-butyldimethylsilyloxy and there is a single bond in 23,24 position; and
$R_{4s}$ is hydroxy and there is a single bond in 10,11 position; or absent and there is a double bond in 10,11 position;

or

$R_{1s}$ is a group (b) wherein $R_6$ is methoxy, or a group (d);
$R_{2s}$ is hydroxy optionally protected by tert-butyldimethylsilyloxy and there is a single bond in 23,24 position; or absent and there is a double bond in 23,24 position; and
$R_{4s}$ is hydroxy and there is a single bond in 10,11 position; and
$R_{3s}$ is ethyl or allyl,

in free form and, where such forms exist, in salt form.

A compound of the invention can be obtained by a process comprising

a) for the preparation of a compound of formula I wherein

$R_1$ is a group (a) as defined above under formula I,
$R_2$ and $R_3$ are as defined above under formula I and
$R_4$ is hydroxy

(i.e. a **compound Ia**),

replacing under simultaneous epimerization the hydroxy group by chlorine, bromine, iodine or azido in a corresponding compound having unprotected hydroxy in 33 position (i.e. a **compound IIa**, of formula IIa

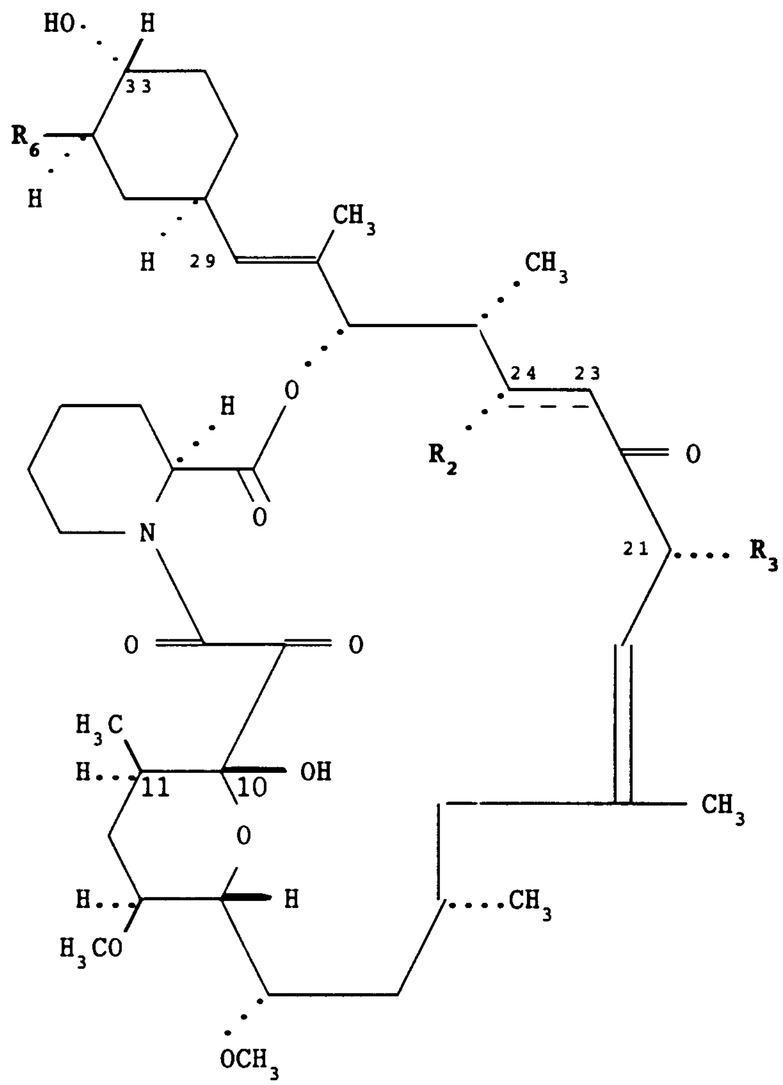

IIa

wherein $R_2$ and $R_3$ are as defined above under formula I and $R_6$ is hydroxy or methoxy);

b) for the preparation of a compound of formula I wherein

| | |
|---|---|
| $R_1$ | is a group (b) as defined above under formula I, |
| $R_2$ and $R_3$ | are as defined above under formula I and |
| $R_4$ | is hydroxy |

(i.e. a **compound Ib**),

treating a corresponding compound IIa with cyanogen bromide in the presence of a base or

treating a corresponding compound IIa with thiophosgene, reacting the resultant product with an inorganic aside and allowing the resultant unstable intermediate having a group

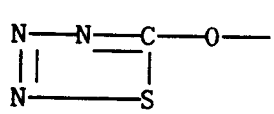

in 33 position (i.e. a **compound IIb**) to decompose to a corresponding compound Ib;

c) for the preparation of a compound of formula I wherein

| | |
|---|---|
| $R_1$ | is a group (d) as defined above under formula I, |
| $R_2$ and $R_3$ | are as defined above under formula I and |
| $R_4$ | is hydroxy |

(i.e. a **compound Ic**),

treating a corresponding compound Ib with an acid having a non-nucleophilic anion; and

- when a resultant compound of formula I has a protected hydroxy and/or a protected amino group,
  optionally splitting off the protecting group(s) to give a corresponding compound of formula I having one or more unprotected hydroxy and/or unprotected amino group(s)
  (i.e. a **compound Ij**),
  whereby when $R_1$ is a group (a), a water molecule may be simultaneously split off and a compound of formula I is obtained wherein
  $R_1$ is a group (a) as defined above under formula I,
  $R_2$ is unprotected hydroxy and there is a single or double bond in 23,24 position; and
  $R_4$ is absent and there is a double bond in 10,11 position
  (i.e. a **compound Ii**); or
- optionally protecting an unprotected hydroxy and/or unprotected amino group in a resultant compound of formula I as appropriate to give a corresponding compound of formula I having one or more protected hydroxy and/or protected amino groups(s) (i.e. a **compound Ik**),

and recovering the resultant compound of formula I in free form and, where such forms exist, in salt form.

The process variants of the invention can be effected in a manner analogous to known procedures.

Process variant a) is a substitution reaction under simultaneous epimerization. It is preferably effected in an inert solvent such as tetrahydrofurane or toluene. Preferably for the substitution by halogen the reaction is effected with tetrachloro-, tetrabromo- or tetraiodomethane in the presence of triphenylphosphine, and for the substitution by azido with azodicarboxylic acid ester, preferably diethyl ester, and hydrazoic acid. A hydroxy group in 24 position may be in protected form. As protecting group known hydroxy protecting groups such as tert-butyldimethylsilyl may be used. A protecting group may subsequently be split off in accordance with known procedures, e.g. with hydrofluoric acid in acetonitrile. Upon deprotection a water molecule may, depending on the reaction conditions chosen, simultaneously be split off in position 10,11 and a double bond formed. The individual compounds can be separated from such a resultant mixture in conventional manner, e.g. chromatographically.

Compounds Ia may be further processed by e.g. oxidation or dehydration to corresponding compounds wherein $R_4$ is absent; for example, oxidation of compounds Ia wherein $R_2$ is hydroxy leads to corresponding compounds wherein $R_4$ is absent and $R_2$ is oxo.

Process variant b) is a cyanidation reaction. It preferably is effected in an inert solvent such as a chlorinated hydrocarbon, e.g. dichloromethane. The temperature preferably is about room temperature. The base is e.g. 4-dimethylaminopyridine.

A compound of formula I obtained accordance to process variants a) and b) above may be isolated from the reaction mixture and purified in accordance with known methods. When $R_2$ is hydroxy and there is a single bond in 23,24 position a water molecule may be simultaneously split off. A corresponding mixture of compounds Ib is obtained wherein either $R_2$ is hydroxy and there is a single bond in 23,24 position or $R_2$ is absent and there is a double bond in 23,24 position. The individual compounds can be separated from such a resultant mixture in conventional manner, e.g. chromatographically.

The second procedure according to process variant b) is effected by reaction with thiophosgene, preferably in the presence of an acid scavenger such as 4-dimethylaminopyridine. Preferably an inert solvent such as acetonitrile is used. The temperature preferably is about room temperature. The subsequent reaction with an inorganic aside is preferably effected with sodium azide. The resultant compounds IIb are unstable and decompose already at room temperature to compounds Ib, under splitting off of nitrogen and sulfur. This reaction step preferably is effected in an inert solvent such as an aromatic hydrocarbon, e.g. benzene. Temperature preferably is elevated, e.g. about 50 °C.

In process variant c) a ring contraction takes place. Protecting groups which are present may be simultaneously split off. Preferably an inert solvent such as acetonitrile is used. Preferably hydrofluoric acid is used as acid having a non-nucleophilic anion. Temperature preferably is about room temperature.

The optional deprotection process variant may also be effect in conventional manner. For splitting off of e.g. tert-butyldimethylsilyl it is effected by treatment with e.g. hydrofluoric acid in a solvent such as acetonitrile. Depending on the reaction conditions selected (duration, temperature, etc.) the splitting can be steered in such a manner that either all or only some protecting group are removed. Partial deprotection is particularly indicated where a definite hydroxy group is to be subsequently reacted in a later reaction.

The optional protection step variant may also be effected in conventional manner along similar lines.

Thus for subsequent reactions involving a hydroxy group, particularly a hydroxy group in position 24 and/or 33, selective protection of only one of the two free hydroxy groups or selective deprotection of only one of the two protected hydroxy groups may be effected in such a manner that reaction occurs only at the desired position. Mixtures of end products may be obtained thereby; such mixtures can be separated in conventional manner, e.g chromatographically. Resultant end products still containing protecting groups can

be subsequently deprotected. Reaction conditions may alternatively be selected such that simultaneously with or immediately after reaction the protecting groups are removed (one pot process).

The compounds of formula I may be isolated and purified from the reaction mixture in conventional manner.

Insofar as their preparation is not specifically described herein, e.g. in the Examples, the compounds used as starting materials are known or can be obtained in conventional manner from known compounds, e.g. starting from appropriate Streptomyces strains such as Streptomyces tsukubaensis No. 9993 described in e.g. Fujisawa EP 184162. Samples can be obtained from the Fermentation Research Institute, Tsukuba, Ibaraki 305, Japan under provisions of the Budapest Treaty under deposit No. FERM BP-927. This strain has been redeposited on April 27, 1989 e.g. as disclosed in Sandoz EP 356399, with the Agricultural Research Culture Collection International Depository, Peoria, Illinois 61604, USA under the provisions of the Budapest Treaty under deposit No. NRRL 18488.

The following prior art has been cited during prosecution of the present filing:

- D1 = H.Tanaka et al., J.Am.Chem.Soc. 109 (1987) 5031-5033;
- D2 = Fujisawa EP-A-0 184 162;
- D3 = Fisons EP-A-0 323 042.

The following Examples illustrate the invention and are not limitative. All temperatures are in degrees Centigrade. All NMR spectra are in $CDCl_3$, ppm. The abbreviations have the following meanings:

| | |
|---|---|
| BOC: | tert-butoxycarbonyl; |
| cfr: | colourless foamy resin: |
| db: | double bond; |
| Et: | ethyl; |
| FK 506: | the compound of formula |

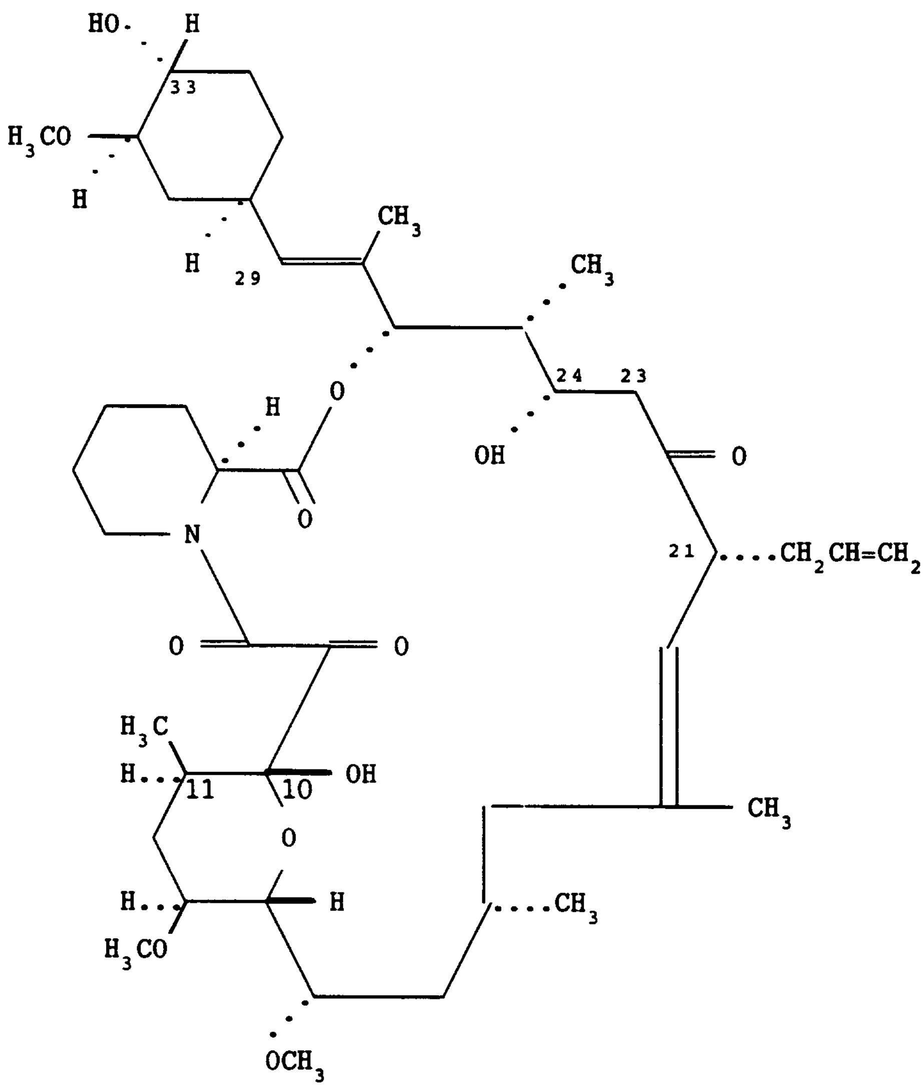

i.e. 17α-allyl-1β,14α-dihydroxy-12-[2'-(4"(R)-hydroxy-3"(R)-methoxycyclohex-1"(R)-yl)-1'-methyl-trans-vinyl]-23α,25β-dimethoxy-13α, 19,21α,27β-tetramethyl-11,28-dioxa-4-azatricyclo[22.3.1.0$^{4,9}$]octacos-18-trans-ene-2,3,10,16-tetraone (according to the atom numbering in EP 184162; however, in the Examples the atom numbering of formula I is used throughout);

| | |
|---|---|
| FR 520: | as FK 506, but with ...$CH_2CH_3$ (ethyl) in place of allyl in position 21 in the formula; |
| iBuoyloxy: | isobutanoyloxy [$(H_3C)_2CHCOO$-]; |
| iPr: | isopropyl; |
| na: | not applicable; |
| $N_3$: | azido; |
| OMe (or MeO): | methoxy; |
| OtBDMS: | tert-butyldimethylsilyloxy; |
| sb: | single bond; |
| tBu: | tert-butyl. |

Examples 15 to 63 illustrate the preparation of compounds no longer encompassed by the scope of the present invention but useful as intermediates for the preparation of compounds of formula I. They are of formula X

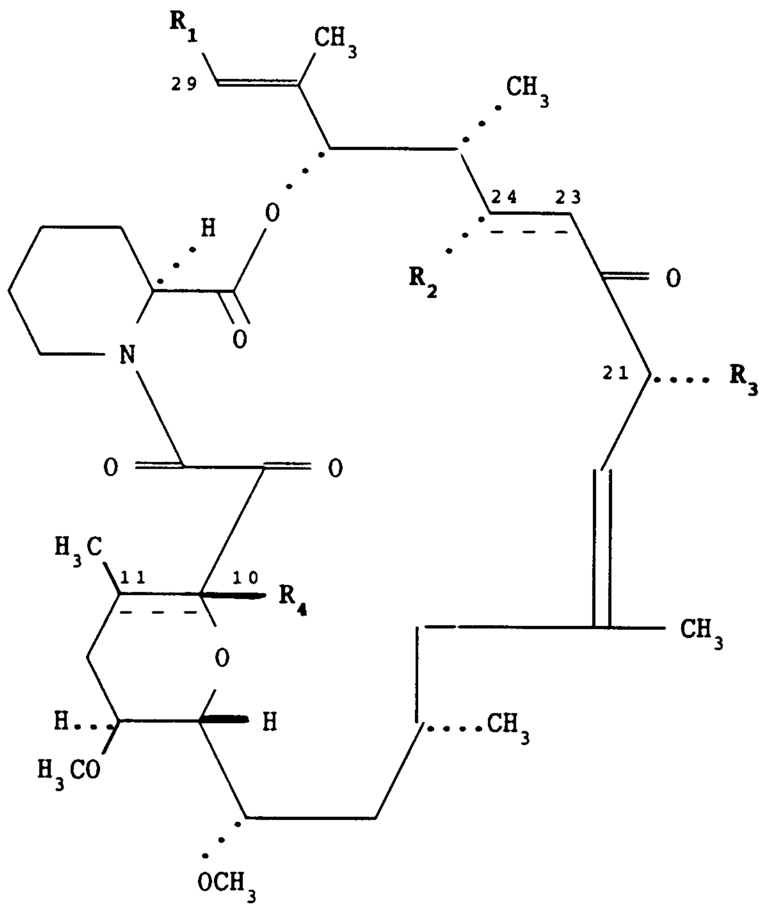

X

wherein

$R_1$ is a group (c) of formula

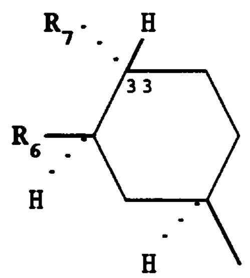

(c)

wherein

$R_6$ is as defined above and

$R_7$ is oxo; optionally protected hydroxy; methoxy; methylthiomethoxy; isobutanoyloxy; aminooxalyloxy; $R_8R_9CRCOO$- wherein $R_8$ is optionally protected hydroxy or optionally protected amino and $R_9$ is hydrogen or methyl; or p-tolyloxythiocarbonyloxy;

$R_2$ is oxo and there is a single bond in 23,24 position; absent and there is a double bond in 23,24 position; or is optionally protected hydroxy, methoxy, methylthiomethoxy, isobutanoyloxy, aminooxalyloxy or $R_8R_9CHCOO$- wherein $R_8$ and $R_9$ are as defined above, and there is a single or a double bond in 23,24 position;

whereby for that group (c)

1) when $R_7$ is oxo, unprotected hydroxy or methoxy then $R_2$ is other than absent and other than unprotected hydroxy or methoxy, and there is a single bond in 23,24 position;

2) when $R_6$ is methoxy and $R_7$ is methylthiomethoxy
then $R_2$ is other than absent and other than unprotected hydroxy;
3) when $R_6$ is methoxy and $R_7$ is protected hydroxy
then $R_2$ is other than optionally protected hydroxy; and
4) when $R_6$ is hydroxy
then $R_7$ is other than optionally protected hydroxy; and

$R_4$ is hydroxy and there is a single bond in 10,11 position; and
$R_3$ is as defined above,

in free form and, where such forms exist, in salt form.

**Example 1: 24-tert-Butyldimethylsilyloxy-33-epi-33-chloro-FK506**

[Formula I: $R_1$ = a group (a) wherein $R_5$ = chloro, $R_6$ = OMe; $R_2$ = OtBDMS, single bond in 23,24 position; $R_3$ = allyl; $R_4$ = OH, single bond in 10,11 position]

[Process variant a), replacement with epimerization]

0.092 g **24-tert-butyldimethylsilyloxy-FK506** is heated for 15 hours under refluxing with 0.037 g triphenylphosphine in 4 ml of tetrachloromethane. The solvent is evaporated to dryness under reduced pressure and the residue is purified by column chromatography over silicagel using a mixture of hexane and acetic acid ethyl ester (2:1) as the eluant. The **title compound** is obtained (colourless foam):

$^1$H-NMR: about 2:3 mixture of conformers:
main conformer: 4.56 (m, $w_{1/2}$ = 7 Hz, H-33).

The starting material is obtained as follows:

a) 20 g **FK 506** is dissolved in 400 ml of dry dimethylformamide, 5.08 g imidazole and 11.25 g tert-butyldimethylchlorosilane is added in portions and the mixture is stirred for 110 hours at room temperature. The reaction mixture is diluted with acetic acid ethyl ester and washed five times with water. The organic phase is dried over sodium sulfate and the solvent distilled off under reduced pressure. The resultant crude product is purified by chromatography over silicagel using hexane/acetic acid ethyl ester 3:1 as the eluant. **24,33-Bis-(tert-butyldimehylsilyloxy)-FK 506** is obtained:

$^{13}$C-NMR: main conformer: 69.7 (C-24); 75.1 (C-33); 84.1 (C-32); 164.6 (C-8); 168.9 (C-1); 196.4 (C-9); 209.3 (C-22);
minor conformer: 70.9 (C-24); 75.3 (C-33); 84.1 (C-32); 165.8 (C-8); 168.2 (C-1); 191.2 (C-9); 210.0 (C-22);

b) 0.5 g 24,33-bis-(tert-butyldimethylsilyloxy)-FK506 is dissolved at 0° under stirring into a mixture of 10 ml of acetonitrile and 0.5 ml of 40 % hydrofluoric acid. After 2 hours at that temperature the reaction medium is diluted with dichloromethane. The solution is successively washed with saturated aqueous sodium bicarbonate solution and water and the organic phase is dried over sodium sulfate, and the solvent is evaporated under reduced pressure. The resultant residue is purified by column chromatography over silicagel (eluant: dichloromethane/methanol 9:1). **24-tert-Butyldimethylsilyloxy-FK 506** is obtained as a colourless foam:

$^{13}$C-NMR: main conformer: 69.7 (C-24); 73.6 (C-33); 84.1 (C-32); 164.6 (C-8); 168.9 (C-1); 196.4 (C-9); 209.2 (C-22);
minor conformer: 70.7 (C-24); 73.6 (C-33); 84.2 (C-32); 165.8 (C-8); 168.2 (C-1); 191.4 (C-9); 209.2 (C-22).

**Example 2: 24-tert-Butyldimethylsilyloxy-33-epi-33-azido-FK506**

[Formula I: $R_1$ = a group (a) wherein $R_5$ = azido, $R_6$ = OMe; $R_2$ = OtBDMS, single bond in 23,24 position; $R_3$ = allyl; $R_4$ = OH, single bond in 10,11 position]

[Process variant a)]

To a solution of 0.092 g **24-tert-butyldimethylsilyloxy-FK506** and 0.08 g triphenylphosphine in 2 ml of dry tetrahydrofurane is added at 0° 0.047 ml of azodicarboxylic acid diethyl ester, followed by 0.15 ml of a 2 M solution of hydrazoic acid in toluene. The solution is brought to room temperature and stirred for 18 hours. The solvent is evaporated to dryness under reduced pressure and the residue purified as described above under Example 1. The **title compound** is obtained (colourless foam):

$^{1}$H-NMR: 4.07 (m, H-33).

The following compounds of formula I are obtained in analogous manner in accordance with process variant a):

| Example No. | Analogous to Ex. No. | $R_1$ | $R_5$ | $R_6$ | $R_7$ | $R_2$ | Position 23,24 | $R_3$ | $R_4$ | Position 10,11 | Physicochemical characterization data |
|---|---|---|---|---|---|---|---|---|---|---|---|
| 3 | 1[1)] | (a) | Cl | OMe | na | OtBDMS | sb | Et | OH | sb | NMR* |
| 4 | 1[1)] | (a) | Br | OMe | na | OtBDMS | sb | Et | OH | sb | |
| 5 | 1[1)] | (a) | Br | OMe | na | OtBDMS | sb | allyl | OH | sb | |
| 6 | 2[1)] | (a) | $N_3$ | OMe | na | OtBDMS | sb | Et | OH | sb | |
| 6a | 1[1)] | (a) | I | OMe | na | OtBDMS | sb | Et | OH | sb | NMR* |

*NMR: Example 3: $^{1}$H-NMR: 4.56 (m, H-33);
Example 6a: $^{13}$C-NMR: mixture of conformers: 210.33 (C-22); 168.91 (C-1); 164.59 (C-8); 123.64 (C-20); 78.90 (C-32); 25.81 (tBu).

[1)] The starting material is obtained from FR 520 in a manner analogous to 24-tert-butyldimethylsilyloxy-FK 506 (see Example 1):

a) **24,33-bis-(tert-butyldimethylsilyloxy)-FR 520:** $^{1}$H-NMR: about 2:1 mixture of 2 conformers:
main conformer: 4.42 (m, H-2); 4.41 (db, 13 Hz, H-6 eq.); 4.05 (txt, J=1.5 Hz and 6 Hz, H-24); 3.80 (dxd, J=1.5 Hz and 10 Hz, H-14); 2.95 (dxdxd, J=4 Hz, 8 Hz and 11 Hz, H-32);
minor conformer: 4.25 (q, J=5 Hz, H-24); 3.94 (dxd, J=2 Hz and 10 Hz, H-14); 2.95 (dxdxd, J=4 Hz, 8 Hz and 11 Hz, H-32);

b) **24-tert-butyldimethylsilyloxy-FR 520:** $^{1}$H-NMR: about 2:1 mixture of 2 conformers:
main conformer: 4.44 (b, H-2); 4.42 (db, J=13 Hz, H-6 eq.); 4.05 (dxt, J=1,5 Hz and 6 Hz, H-24); 3.81 (dxd, J=1.5 Hz and 10 Hz, H-14); 3.01 (dxdxd, J=4 Hz, 8 Hz and 11 Hz, H-32);
minor conformer: 4.24 (H-24); 3.94 (dxd, J=2 Hz and 10 Hz, H-14); 3.01 (dxdxd, J=4 Hz, 8 Hz and 11 Hz, H-32).

**Example 7: 24-tert-Butyldimethylsilyloxy-33-cyanoxy-FR 520**

[Formula I: $R_1$ = a group (b) wherein $R_6$ = OMe; $R_2$ = OtBDMS, single bond in 23,24 position; $R_3$ = Et; $R_4$ = OH, single bond in 10,11 position]

[Process variant b), treatment with cyanogen bromide]

A solution of 2 g **24-tert-butyldimethylsilyloxy-FR 520** and 0.94 g 4-dimethylaminopyridine in 100 ml of dichloromethane is rapidly reacted at room temperature with a solution of 0.4 g cyanogen bromide in 15 ml of dichloromethane and the mixture is stirred at room temperature for 20 minutes. The mixture is filtered over silicagel (eluant: n-hexane/acetic acid ethyl ester) and the solvent is removed from the relevant fraction under reduced pressure. The **title compound** is obtained as a colourless foamy resin:

$^1$H-NMR: mixture of conformers: 4.3 (m; H-33).

**Example 8: 24-tert-Butyldimethylsilyloxy-33-cyanoxy-FR 520**

[Formula I: as for Example 7]

[Process variant b), treatment with thiophosgene and sodium azide]

A solution of 2 g **24-tert-butyldimethylsilyloxy-FR 520** and 2 g 4-dimethylaminopyridine in 50 ml of acetonitrile is carefully reacted with 0.4 ml of thiophosgen and the mixture stirred for 3 hours at room temperature. The reaction mixture is poured onto a well-stirred mixture consisting of 150 ml of acetic acid ethyl ester, 40 ml of saturated aqueous sodium chloride solution and 50 ml of 2 N sodium azide solution, vigourous stirring is continued for 5 minutes and the organic phase is separated. The organic phase is then successively washed with water, 1 N hydrochloric acid solution, water, and saturated aqueous sodium chloride solution, dried over sodium sulfate, filtered and concentrated under reduced pressure. The residue is taken up in about 100 ml of benzene and heated at 30-40° for 2 hours. The benzene is removed under reduced pressure and the **title compound** is recovered from the residue as a colourless foamy resin by column chromatography over silicagel (eluant: n-hexane / acetic acid ethyl ester):

$^1$H-NMR: see Example 7.

The following compounds of formula I are obtained in analogous manner in accordance with process variant b):

| Example No. | Analogous to Ex. No. | $R_1$ | $R_5$ | $R_6$ | $R_7$ | $R_2$ | Position 23,24 | $R_3$ | $R_4$ | Position 10,11 | Physicochemical characterization data |
|---|---|---|---|---|---|---|---|---|---|---|---|
| 9 | 7,8 | (b) | na | OMe | na | OtBDMS | sb | allyl | OH | sb | NMR* |
| 10a[1)] | 7,8 | (b) | na | OMe | na | OH | sb | Et | OH | sb | NMR* |
| 10b[1)] | 7,8 | (b) | na | OMe | na | absent | db | Et | OH | sb | NMR* |
| 11a[2)] | 7,8 | (b) | na | OMe | na | OH | sb | allyl | OH | sb | |
| 11b[2)] | 7,8 | (b) | na | OMe | na | absent | db | allyl | OH | sb | |

*NMR: Example 9: $^{1}$H-NMR: mixture of conformers: 4.3 (m, H-33);

Example 10a: $^{1}$H-NMR: mixture of conformers: 5.34 (H-26); 4.63 (db, J=4 Hz, H-2); 4.44 (db, J=13 Hz, H-6 eq.); 4.30 (dxdxd, J=5 Hz, 8 Hz and 11 Hz, H-33); 3.01 (tb, J=13 Hz, H-6ax.);

Example 10b: $^{1}$H-NMR: 6.81 resp. 6.75 (dxd resp. dxd, J=5 Hz and 15 Hz resp. 7 Hz and 15 Hz, H-24); 6.2 resp. 6.3 (dxd resp. dxd, J=2 Hz and 15 Hz resp. 1 Hz and 15 Hz, H-23); 5.29 resp. 5.23 (d resp. d, J=3 Hz resp. 3 Hz, H-26); 4.3 (m, H-33);

[1)][2)] A mixture of both compounds is obtained; they can be separated chromatographically (eluant: n-hexane/acetic acid ethyl ester).

**Example 12: 29-Des-(4-hydroxy-3-methoxycyclohexyl)-29-(3-formylcyclopentyl)-FR 520**

[Formula I: $R_1$ = a group (d); $R_2$ = OH, single bond in 23,24 position; $R_3$ = Et; $R_4$ = OH, single bond in 10,11 position]

[Process variant c), treatment with a non-nucleophilic anion]

0.5 g **24-tert-butyldimethylsilyloxy-33-cyanoxy-FK 520** (compound of Examples 7 and 8) or **33-cyanory-FR 520** (compound of Example 10a) is dissolved into a mixture of 50 ml of acetonitrile and 2 ml of 40 % wt. aqueous hydrofluoric acid and the mixture is stirred for 2.5 hours at room temperature. The reaction mixture is then distributed between acetic acid ethyl ester and saturated aqueous sodium bicarbonate solution, the aqueous phase is discarded and the organic phase is washed with saturated aqueous sodium chloride solution, dried over sodium sulfate, filtered and concentrated under reduced pressure. The **title compound** is obtained as a colourless foamy resin from the residue by column chromatography over silicagel (eluant: n-hexane / acetic acid ethyl ester):

$^1$H-NMR: mixture of conformers: 9.64 (d, J = 2 Hz, CHO); 2.87 (m, H-32); 2.67 (m, H-30).

The following compounds of formula I are obtained in analogous manner in accordance with process variant c):

| Example No. | Analogous to Ex. No. | $R_1$ | $R_5$ | $R_6$ | $R_7$ | $R_2$ | Position 23,24 | $R_3$ | $R_4$ | Position 10,11 | Physicochemical characterization data |
|---|---|---|---|---|---|---|---|---|---|---|---|
| 13 | 12[1)] | (d) | na | na | na | OH | sb | allyl | OH | sb | NMR* |
| 14 | 12[2)] | (d) | na | na | na | absent | db | Et | OH | sb | NMR* |

*NMR: Example 13: $^1$H-NMR: mixture of conformers: 9.65 (d, J=2 Hz, CHO); 2.86 (m, H-32); 2.15 (dxdxd, J=12.5 Hz and 7.5 Hz and 5 Hz, H-31a); 1.45 (dxt, J=12.5 and 9 Hz, H-31b); 2.67 (m, H-30);

Example 14: $^1$H-NMR: about 5:3 mixture of conformers: 9.66 (d, J=2 Hz, CHO); 6.83 (dxd, J=15 and 5 Hz) resp. 6.77 (dxd, J=15 and 7.5 Hz) H-24; 6.19 (dxd, J=15 and 1.5 Hz) resp. 6.30 (dxd, J=15 and 1 Hz) H-23;

1) Starting from the compound of Example 9 or 11a;
2) Starting from the compound of Example 10b.

**Example 15: a) 24-tert-Butyldimethylsilyloxy-33-oxo-FK506** and b) **24-tert-Butyldimethylsilyloxy-33-methylthiomethoxy FK 506**

[Formula X: $R_1$ = a group (c) wherein $R_6$ = OMe, $R_7$ = oxo and, respectively, methylthiomethoxy; $R_2$ = OtBDMS, single bond in 23,24 position; $R_3$ = allyl; $R_4$ = OH, single bond in 10,11 position]

1 g **24-tert-Butyldimethylsilyloxy-FK 506** is dissolved at room temperature into a mixture of 20 ml of acetic anhydride and 30 ml of dimethylsulfoxide and stirring is effected for 5 hours at room temperature. The reaction mixture is poured onto a mixture of acetic acid ethyl ester and potassium carbonate solution, stirred for 20 minutes, the phases are separated and the organic phase is repeatedly washed with water, dried over sodium sulfate, filtered and concentrated under reduced pressure. Following column chromatographic fractionation of the residue over silicagel (eluant: acetic acid ethyl ester / n-hexane 2:1) the **title compounds** are obtained as colourless foamy resins.

The following compounds of formula X are obtained in analogous manner as colourless foamy resins:

| Example No. | Analogous to Ex. No. | $R_1$ | $R_5$ | $R_6$ | $R_7$ | $R_2$ | Position 23,24 | $R_3$ | $R_4$ | Position 10,11 |
|---|---|---|---|---|---|---|---|---|---|---|
| 16a | 15[1)] | (c) | na | OMe | OtBDMS | $OCH_2SCH_3$ | sb | allyl | OH | sb |
| 16b | 15[1)] | (c) | na | OMe | OtBDMS | oxo | sb | allyl | OH | sb |
| 16c | 15[2)] | (c) | na | OMe | oxo | OtBDMS | sb | Et | OH | sb |
| 16d | 15[3)] | (c) | na | OMe | OtBDMS | oxo | sb | Et | OH | sb |

1) Starting from **33-tert-butyldimethylsilyloxy-FK 506** (compound of Example 16 in EP 184162);
eluant: toluene / acetic acid ethyl ester 9:1;

2) Starting from **24-tert-butyldimethylsilyloxy-FR 520**;

3) Starting from **33-tert-butyldimethylsilyloxy-FR 520** (DOS 39 38 754);

**Example 17: 33-p-Tolyloxythiocarbonyloxy-FK506**

[Formula X: $R_1$ = a group (c) wherein $R_6$ = OMe, $R_7$ = p-tolyloxythiocarbonyloxy; $R_2$ = OH, single bond in 23,24 position; $R_3$ = allyl; $R_4$ = OH, single bond in 10,11 position]

A solution of 2 g **FK 506** in 70 ml of acetonitrile is successively reacted with 0.46 g 4-dimethylaminopyridine and 1.8 g p-tolyloxythiocarbonyl chloride and the mixture is stirred for 15 hours at room temperature. The reaction mixture is then diluted with acetic acid ethyl ester and successively washed with saturated aqueous sodium bicarbonate solution, 0.5 N hydrochloric acid and water, dried over sodium sulfate, filtered and concentrated under reduced pressure. The **title compound** is isolated from the residue as a light yellow foamy resin by column chromatography over silicagel (eluant: acetic acid ethyl ester / n-hexane 1:1).

**Example 18: 33-Aminomethylcarbonyloxy-$\Delta^{23}$ -FK506**

[Formula X: $R_1$ = a group (c) wherein $R_6$ = OMe, $R_7$ = $R_8R_9CHCOO$-($R_8$ = amino; $R_9$ = H); $R_2$ = absent, double bond in 23,24 position; $R_3$ = allyl; $R_4$ = OH, single bond in 10,11 position]

2 g N-BOC-glycine, 1 g dicyclohexylcarbodiimide, 0.5 g **$\Delta^{23}$ -FK 506** (second compound of Example 17 in EP 184162) and 1 g 4-dimethylaminopyridine are successively taken up at room temperature in 70 ml of acetonitrile and the mixture is stirred for 20 minutes at room temperature. The reaction mixture is filtered, the filtrate diluted with acetic acid ethyl ester and successively washed with 1 N hydrochloric acid, aqueous sodium bicarbonate solution and water, the organic phase is dried over sodium sulfate, filtered, concentrated, and the **residue** is taken up in 50 ml of acetonitrile.

In order to split off the protecting group 0.5 g p-toluenesulfonic acid monohydrate is added and the mixture heated to refluxing for 5 minutes, the solution is cooled off, diluted with acetic acid ethyl ester, washed to neutrality with water, the organic phase is dried over sodium sulfate and concentrated. From the residue the **title compound** is obtained as a colourless foamy resin after column chromatography over silicagel (eluant: acetic acid ethyl ester / methanol 20:3).

**Example 19: 24-tert-Butyldimethylsilyloxy-FR 520-33-[(tert-butyldimethylsilyloxy)-(S)-lactate]**

[Formula X: $R_1$ = a group (c) wherein $R_6$ = OMe, $R_7$ = $R_8R_9CHCOO$-($R_8$ = OtBDMS, $R_9$ = Me, S-configuration); $R_2$ = OtBDMS, single bond in 23,24 position; $R_3$ = Et; $R_4$ = OH, single bond in 10,11 position]

To a solution of 450 mg **24-tert-butyldimethylsilyloxy-FR 520** and 120 mg tert-butyldimethylsilyloxy-(S)-lactic acid in 10 ml of dichloromethane are added at room temperature 120 mg N-ethyl-N'-(3-dimethylaminopropyl)carbodiimide hydrochloride and 23 mg dimethylaminopyridine. After 60 hours the reaction mixture is diluted with acetic acid ethyl ester, washed successively with 0.5 N hydrochloric acid and then water, dried over sodium sulfate, filtered, and the solvent is evaporated under reduced pressure. The residue is chromatographed over silicagel (eluant: n-hexane / acetic acid ethyl ester 2:1). The **title compound** is obtained as a colourless foam.

**Example 20: FK 506-33-glycolate**

[Formula X: $R_1$ = a group (c) wherein $R_6$ = OMe, $R_7$ = $R_8R_9CHCOO$- ($R_8$ = OH, $R_9$ = H); $R_2$ = OH, single bond in 23,24 position; $R_3$ = allyl; $R_4$ = OH, single bond in 10,11 position]

To a solution of 300 mg tert-butyldimethylsilyloxymethylcarboxylic acid in 5 ml of dichloromethane are added under stirring at 0° 0.67 ml of oxalyl chloride and one drop of dimethylformamide. The mixture is brought to room temperature and is stirred for 1 hour. The reaction mixture is concentrated under reduced pressure. The residue is taken up in 5 ml of dichloromethane and this solution is added dropwise at 0° to a solution of 600 mg **FK 506,** 0.28 ml triethylamine and a catalytic quantity of 4-dimethylaminopyridine. After 18 hours stirring at 0° the solution is diluted with acetic acid ethyl ester, successively washed with 0.1 N hydrochloric acid and water, the organic phase is dried over sodium sulfate, filtered and concentrated under reduced pressure. The residue is taken up in 20 ml of acetonitrile, reacted with 0.5 ml of 40 % wt. aqueous hydrofluoric acid and stirred for 20 minutes at room temperature. The mixture is diluted with acetic acid

ethyl ester, washed with saturated aqueous sodium hydrogen carbonate solution, dried over sodium sulfate, filtered and concentrated under reduced pressure. The **title compound** is obtained as a colourless foamy resin from the residue by chromatography over silicagel (eluant: n-hexane / acetic acid ethyl ester).

The following compounds of formula X are obtained in analogous manner as a colourless foamy resin:

| Example No. | Analogous to Ex. No. | $R_1$ | $R_5$ | $R_6$ | $R_7$ | $R_2$ | Position 23,24 | $R_3$ | $R_4$ | Position 10,11 |
|---|---|---|---|---|---|---|---|---|---|---|
| 21 | 17 to 20 | (c) | na | OMe | BOC-$NHCH_2COO$- | OtBDMS | sb | allyl | OH | sb |
| 22 | 17 to 20 | (c) | na | OMe | tBDMS-$OCH_2COO$- | OtBDMS | sb | allyl | OH | sb |
| 23 | 17 to 20 | (c) | na | OMe | BOC-$NHCH_2COO$- | OtBDMS | sb | Et | OH | sb |
| 24 | 17 to 20 | (c) | na | OMe | tBDMS-$OCH_2COO$- | OtBDMS | sb | Et | OH | sb |
| 25a | 17 to 20 | (c) | na | OMe | BOC-$NHCH_2COO$- | OH | sb | allyl | OH | sb |
| 25b | 17 to 20 | (c) | na | OMe | OH | BOC-$NHCH_2COO$- | sb | allyl | OH | sb |
| 25c | 17 to 20 | (c) | na | OMe | BOC-$NHCH_2COO$- | BOC-$NHCH_2COO$- | sb | allyl | OH | sb |
| 25d | 17,19,20 | (c) | na | OMe | BOC-$NHCH_2COO$- | absent | db | allyl | OH | sb |
| 26a | 17 to 20 | (c) | na | OMe | BOC-$NHCH_2COO$- | OH | sb | Et | OH | sb |
| 26b | 17 to 20 | (c) | na | OMe | OH | BOC-$NHCH_2COO$- | sb | Et | OH | sb |
| 26c | 17 to 20 | (c) | na | OMe | BOC-$NHCH_2COO$- | BOC-$NHCH_2COO$- | sb | Et | OH | sb |
| 26d | 17 to 20 | (c) | na | OMe | BOC-$NHCH_2COO$- | absent | db | Et | OH | sb |
| 27a | 17 to 20 | (c) | na | OMe | $NH_2COCOO$- | OH | sb | allyl | OH | sb |
| 27b | 17 to 20 | (c) | na | OMe | $NH_2COCOO$- | $NH_2COCOO$- | sb | allyl | OH | sb |
| 27c | 17 to 20 | (c) | na | OMe | $NH_2COCOO$- | absent | db | allyl | OH | sb |
| 28a | 17 to 20 | (c) | na | OMe | $NH_2COCOO$- | OH | sb | Et | OH | sb |
| 28b | 17 to 20 | (c) | na | OMe | $NH_2COCOO$- | $NH_2COCOO$- | sb | Et | OH | sb |
| 28c | 17 to 20 | (c) | na | OMe | $NH_2COCOO$- | absent | db | Et | OH | sb |
| 29 | 17 to 20 | (c) | na | OMe | $NH_2COCOO$- | OtBDMS | sb | Et | OH | sb |
| 30 | 17 to 20 | (c) | na | OMe | $NH_2COCOO$- | OtBDMS | sb | allyl | OH | sb |
| 31 | 17 to 20 | (c) | na | OMe | p-tolyloxy-thiocarbonyloxy | OH | sb | Et | OH | sb |
| 32 | 17 to 20 | (c) | na | OMe | $HOCH_2COO$- | OH | sb | Et | OH | sb |
| 33 | 17 to 20 | (c) | na | OMe | tBDMS-$OCH(CH_3)COO$-(S) | OH | sb | Et | OH | sb |
| 34 | 17 to 20 | (c) | na | OMe | iBuoyloxy | OtBDMS sb | Et | OH | sb | |
| 35 | 17 to 20 | (c) | na | OMe | iBuoyloxy | OtBDMS sb | allyl | OH | sb | |
| 36 | 17 to 20 | (c) | na | OMe | iBuoyloxy | OH | sb | allyl | OH | sb |
| 37 | 17 to 20 | (c) | na | OMe | iBuoyloxy | OH | sb | Et | OH | sb |
| 38 | 17,18,20 | (c) | na | OMe | tBDMS-$OCH(CH_3)COO$-(S) | OtBDMS | sb | Et | OH | sb |

**Example 39: 24-tert-Butyldimethylsilyloxy-33-aminooxalyloxy-FK506**

[Formula X: $R_1$ = a group (c) wherein $R_6$ = OMe, $R_7$ = aminooxalyloxy; $R_2$ = OtBDMS, single bond in 23,24 position; $R_3$ = allyl; $R_4$ = OH, single bond in 10,11 position]

A solution of **24,33-bis(tert-butyldimethylsilyloxy)-FK 506** in 70 ml of acetonitrile is reacted at 0° to 5° with 1 ml of oxalyl chloride and stirred at 0 to 5° for 40 minutes. The reaction mixture is stirred with a mixture of acetic acid ethyl ester and satured aqueous ammonia solution, any precipitate formed is sucked off, the phases are separated, the organic phase is washed successively with 1 N hydrochloric acid and then water, dried over sodium sulfate, filtered and concentrated under reduced pressure. From the residue the **title compound** is obtained as a colourless foamy resin following column chromatography over silicagel (eluant: n-hexane / acetic acid ethyl ester 1:1).

The following compounds of formula X are obtained in analogous manner as a colourless foamy resin:

| Example No. | Analogous to Ex. No. | $R_1$ | $R_5$ | $R_6$ | $R_7$ | $R_2$ | Position 23,24 | $R_3$ | $R_4$ | Position 10,11 |
|---|---|---|---|---|---|---|---|---|---|---|
| 40 | 39[1)] | (c) | na | OMe | $NH_2COCOO-$ | $NH_2COCOO-$ | sb | allyl | OH | sb |
| 41 | 39,40 | (c) | na | OMe | $NH_2COCOO-$ | OtBDMS | sb | Et | OH | sb |
| 42 | 39,40 | (c) | na | OMe | $NH_2COCOO-$ | $NH_2COCOO-$ | sb | Et | OH | sb |

[1)] Stirring is effected for 1 hour at room temperature; column chromatography is effected using an eluant gradient of 3:1 to 1:3.

**Example 43: 24-Methoxy-33-tert-butyldimethylsilyloxy-FK506**

[Formula X: $R_1$ = a group (c) wherein $R_6$ = OMe, $R_7$ = OtBDMS; $R_2$ = OMe, single bond in 23,24 position; $R_3$ = allyl; $R_4$ = OH, single bond in 10,11 position]

1 g **33-tert-butyldimethylsilyloxy-FK 506** is dissolved into a mixture of 50 ml of dichloromethane and 0.04 ml of borotrifluoride etherate previously cooled to 0° to 5°. A solution of 20 ml of an approximately 1 N solution of diazomethane in methylene chloride is then added dropwise in such a manner that the yellow coloration of the solution which initially forms persists for as shortly as possible. The reaction mixture is diluted with acetic acid ethyl ester, successively washed with saturated aqueous sodium hydrogen carbonate solution and water, dried over sodium sulfate, filtered and the solvent is removed under reduced pressure. The **title compound** is obtained as a colourless foamy resin from the residue following column chromatographic purification over silicagel (eluant: acetic acid ethyl ester / n-hexane).

The following compounds of formula X are obtained in analogous manner as a colourless foamy resin:

| Example No. | Analogous to Ex. No. | $R_1$ | $R_5$ | $R_6$ | $R_7$ | $R_2$ | Position 23,24 | $R_3$ | $R_4$ | Position 10,11 |
|---|---|---|---|---|---|---|---|---|---|---|
| 44 | 43[1)] | (c) | na | OMe | OMe | OtBDMS | sb | allyl | OH | sb |

1) Starting from 24-tert-butyldimethylsilyloxy-FK 506.

**Example 45: 24-tert-Butyldimethylsilyloxy-33-oxo-FR 520**

[Formula X: $R_1$ = a group (c) wherein $R_6$ = OMe, $R_7$ = oxo; $R_2$ = OtBDMS, single bond in 23,24 position $R_3$ = Et; $R_4$ = OH, single bond in 10,11 position]

2 g **24-tert-butyldimethylsilyloxy-FR 520** and 1 g N-methylmorpholin-N-oxide are dissolved in 100 ml of methylene chloride, reacted with 5 g molecular sieve (Molsieb 4A) and the mixture is stirred for 15 minutes at room temperature. 0.15 g tetrapropylammonium perruthenate is added and stirring is continued for 3 more hours at room temperature. The mixture is concentrated, the residue is taken up in acetic acid ethyl ester and the solution successively washed with saturated aqueous sodium hydrogen sulfite solution, saturated aqueous sodium chloride and saturated aqueous copper sulfate solution, the organic phase is dried over sodium sulfate, filtered and concentrated under reduced pressure. The **title compound** is obtained from the residue following column chromatography over silicagel (eluant: n-hexane / acetic acid ethyl ester).

The following compounds of formula X are obtained in analogous manner as a colourless foamy resin:

| Example No. | Analogous to Ex. No. | $R_1$ | $R_5$ | $R_6$ | $R_7$ | $R_2$ | Position 23,24 | $R_3$ | $R_4$ | Position 10,11 |
|---|---|---|---|---|---|---|---|---|---|---|
| 46 | 45[1)] | (c) | na | OMe | OtBDMS | oxo | sb | Et | OH | sb |
| 46a | 45[2)] | (c) | na | OMe | oxo | OtBDMS | sb | allyl | OH | sb |
| 46b | 45 | (c) | na | OMe | OtBDMS | oxo | sb | allyl | OH | sb |

1) Starting from 33-tert-butyldimethylsilyloxy-FR 520 (DOS 39 38 754);
2) Starting from 24-tert-butyldimethylsilyloxy-FK 506.

**Example 47: 24-Oxo-FK 506**

[Formula X: $R_1$ = a group (c) wherein $R_6$ = OMe, $R_7$ = OH; $R_2$ = oxo, single bond in 23,24 position; $R_3$ = allyl; $R_4$ = OH, single bond in 10,11 position]

3.6 g **24-oxo-33-tert-butyldimethylsilyloxy-FK506** (compound of Example 16b) is dissolved at room temperature into a mixture of 110 ml of acetonitrile and 3 ml of 40 % wt. aqueous hydrofluoric acid and the mixture is stirred at room temperature for 45 minutes. The reaction mixture is diluted with acetic acid ethyl ester, washed successively with saturated aqueous sodium bicarbonate solution and then water, dried over sodium sulfate, filtered and concentrated under reduced pressure. The **title compound** is obtained as a colourless foamy resin following chromatographic purification of the residue over silicagel (eluant: acetic acid ethyl ester / n-hexane 3:2).

The following compounds of formula X are obtained in analogous manner as a colourless foamy resin:

| Example No. | Analogous to Ex. No. | $R_1$ | $R_5$ | $R_6$ | $R_7$ | $R_2$ | Position 23,24 | $R_3$ | $R_4$ | Position 10,11 |
|---|---|---|---|---|---|---|---|---|---|---|
| 48 | 47[1)] | (c) | na | OMe | $NH_2CH_2COO-$ | OH | sb | allyl | OH | sb |
| 49 | 47[2)] | (c) | na | OMe | $NH_2CH_2COO-$ | absent | db | allyl | OH | sb |
| 50 | 47[3)] | (c) | na | OMe | OH | oxo | sb | Et | OH | sb |
| 51 | 47[4)] | (c) | na | OMe | $NH_2CH_2COO-$ | OH | sb | Et | OH | sb |
| 52 | 47[5)] | (c) | na | OMe | $NH_2CH_2COO-$ | absent | db | Et | OH | sb |
| 53 | 47[6)] | (c) | na | OMe | $HOCH_2COO-$ | OH | sb | allyl | OH | sb |
| 54 | 47[7)] | (c) | na | OMe | $HOCH_2COO-$ | OH | sb | Et | OH | sb |
| 55 | 47[8)] | (c) | na | OMe | $HOCH(CH_3)COO-$ (S) | OH | sb | Et | OH | sb |
| 56 | 47[9)] | (c) | na | OMe | OH | $NH_2CH_2COO-$ | sb | Et | OH | sb |
| 57 | 47[10)] | (c) | na | OMe | $NH_2CH_2COO-$ | $NH_2CH_2COO-$ | sb | Et | OH | sb |
| 58 | 47[11)] | (c) | na | OMe | $NH_2CH_2COO-$ | $NH_2CH_2COO-$ | sb | allyl | OH | sb |
| 59 | 47[12)] | (c) | na | OMe | OH | $NH_2CH_2COO-$ | sb | allyl | OH | sb |
| 60 | 47[13)] | (c) | na | OMe | $NH_2CH_2COO-$ | OH | sb | allyl | OH | sb |
| 61 | 47[14)] | (c) | na | OMe | $NH_2CH_2COO-$ | OH | sb | Et | OH | sb |
| 62 | 47[15)] | (c) | na | OMe | iBuoyloxy | OH | sb | Et | OH | sb |
| 63 | 47[16)] | (c) | na | OMe | iBuoyloxy | OH | sb | allyl | OH | sb |

The following compounds of formula I are obtained in analogous manner in accordance with process variant deprotection:

| Example No. | Analogous to Ex. No. | $R_1$ | $R_5$ | $R_6$ | $R_7$ | $R_2$ | Position 23,24 | $R_3$ | $R_4$ | Position 10,11 | Physicochemical characterization data |
|---|---|---|---|---|---|---|---|---|---|---|---|
| 64 | 47[17)] | (a) | I | OMe | na | OH | sb | Et | OH | sb | cfr; ** |
| 65a | 47[18)] | (a) | Cl | OMe | na | OH | sb | allyl | OH | sb | cfr; NMR* |
| 65b | 47[18)] | (a) | Cl | OMe | na | OH | sb | allyl | absent | db | cfr |
| 66a | 47[19)] | (a) | Cl | OMe | na | OH | sb | Et | OH | sb | cfr; NMR* |
| 66b | 47[19)] | (a) | Cl | OMe | na | OH | sb | Et | absent | db | cfr; NMR* |
| 67a | 47[20)] | (a) | Br | OMe | na | OH | sb | Et | OH | sb | cfr; NMR* |
| 67b | 47[20)] | (a) | Br | OMe | na | OH | sb | Et | absent | db | cfr |
| 68a | 47[21)] | (a) | $N_3$ | OMe | na | OH | sb | allyl | OH | sb | cfr; NMR* |
| 68b | 47[21)] | (a) | $N_3$ | OMe | na | OH | sb | allyl | absent | db | cfr; NMR* |
| 69a | 47[22)] | (a) | Br | OMe | na | OH | sb | allyl | OH | sb | cfr |
| 69b | 47[22)] | (a) | Br | OMe | na | OH | sb | allyl | absent | db | cfr |
| 70a | 47[23)] | (a) | $N_3$ | OMe | na | OH | sb | Et | OH | sb | cfr; NMR* |
| 70b | 47[23)] | (a) | $N_3$ | OMe | na | OH | sb | Et | absent | db | cfr |

1) Starting from the compound of Example 25a;
2) Starting from the compound of Example 25d;
3) Starting from the compound of Example 46 (=16d);
4) Starting from the compound of Example 23;
5) Starting from the compound of Example 26d;
6) Starting from the compound of Example 22;
7) Starting from the compound of Example 24;
8) Starting from the compound of Example 19 or of Example 33;
9) Starting from the compound of Example 26b;

[10] Starting from the compound of Example 26c;
[11] Starting from the compound of Example 25c;
[12] Starting from the compound of Example 25b;
[13] Starting from the compound of Example 25a;
[14] Starting from the compound of Example 26a;
[15] Starting from the compound of Example 34;
[16] Starting from the compound of Example 35;
[17] Starting from the compound of Example 6a;
[18] Starting from the compound of Example 1;
[19] Starting from the compound of Example 3;
[20] Starting from the compound of Example 4;
[21] Starting from the compound of Example 2;
[22] Starting from the compound of Example 5;
[23] Starting from the compound of Example 6;

*NMR:

Example 48: $^1$H-NMR: about 2:1 mixture of conformers:
5.33 and 5.20 (d/d, J=1 Hz and 1 Hz, H-26); 4.84 (dxdxd, J=5 Hz, 9.5 Hz and 11 Hz, H-33); 3.44 (s, 2H, $O=C-CH_2-N-$); 3.22 (dxdxd, J=5 Hz, 9.5 Hz and 11 Hz, H-32);

Example 49: $^1$H-NMR: see Example 18;

Example 50: $^1$H-NMR: mixture of conformers:
5.8 and 5.6 (s/s, H-23); 5.69 (H-26); 4.38 (d, J=13 Hz, H-6e); 4.19 (t, H-2); 3.80 (dxd, J=9 Hz and 2 Hz, H-14);

Example 51: $^1$H-NMR: about 2:1 mixture of conformers:
5.34 (d, J=2 Hz, H-26); 4.75 (dxdxd, J=5 Hz, 9 Hz and 10 Hz, H-33); 4.61 (db, J=4 Hz, H-2); 4.44 (db, J=13 Hz, H-6e); 3.45 (s, $-CH_2-N$);

Example 53: $^{1}$H-NMR: see Example 20;

Example 54: $^{1}$H-NMR: see Example 32;

Example 55: $^{1}$H-NMR: mixture of conformers: main conformer: 1.23 (d, J=7 Hz); 4.30 [dq, $J_1$=5 Hz, $J_2$=7 Hz, -COCH($CH_3$)OH]; 4.44 (d, br, J=13 Hz, H-6e); 4.61 (d, br, J=4 Hz); 4.78 (ddd, $J_1$=5 Hz, $J_2$=5 Hz, $J_3$=11 Hz, H-33); 5.34 (H-26);

Example 60: $^{1}$H-NMR: see Example 48;

Example 61: $^{1}$H-NMR: see Example 51;

Example 62: $^{1}$H-NMR: see Example 37;

Example 65a: $^{1}$H-NMR 4.59 (m, H-33);

$^{13}$C-NMR: about 2:3 mixture of conformers:
main conformer: 59.1 (C-33; 79.2 (C-32); 97.5 (C-10); 116.4 (C-38); 123.0 (C-20); 135.6 (C-37); 138.4 (C-19); 164.6 (C-8); 168.9 (C-1); 196.4 (C-9); 209.4 (C-22);

Example 66a: $^{1}$H-NMR: 4.56 (m, H-33);

Example 66b: $^{1}$H-NMR: 2.09 (s, 11-$CH_3$); 4.5 (bm, H-33);

$^{13}$C-NMR: about 2:1 mixture of conformers:
main conformer: 56.2 (C-33); 80.6 (C-32); 116.4 (C-38); 122.9 (C-20); 124.8 (C-11); 129.5 (C-29); 131.9 (C-28): 135.8 (C-37); 140.0 (C-19); 142.9 (C-10); 166.7 (C-8); 168.7 (C-1); 188.0 (C-9); 212.4 (C-22);
minor conformer: 56.1 (C-33); 80.6 (C-32); 116.5 (C-38); 123.6 (C-20); 126.4 (C-11); 128.5 (C-29); 131.8 (C-28); 135.6 (C-37); 137.4 (C-19); 144.1 (C-10); 166.5 (C-8); 169.5 (C-1); 184.8 (C-9); 213.3 (C-22);

Example 67a: $^{1}$H-NMR: 4.44 (d, J=13 Hz, H-6 eq.); 4.60 (d, J=4 Hz, H-2); 4.70 (sb, H-33);

Example 68a: $^{1}$H-NMR: 4.07 (m, $w_{1/2}$ = 8 Hz, H-33);

Example 68b: $^{1}$H-NMR: about 2:1 mixture of conformers: 4.06 (m, H-33); 2.09 and 1.94 (2s, 11-$CH_3$);

Example 70a: $^{1}$H-NMR: about 5:4 mixture of conformers: 5.60 resp. 5.79 (s resp. s, H-23); 5.70 resp. 5.66 (d, J=3 Hz resp. d, J=3 Hz, H-26); 4.38 (d, J=13 Hz, H-6e); 4.15 (t, H-2); 3.80 (dxd, J=9 Hz and 2 Hz, H-14).

** Iodine analyis: theor.: 14.06 %; found: 13.57 %.

The compounds of Examples 10a, 11a, 12 and 13 may be prepared in analogous manner according to process variant deprotection.

**Example 71: 24-tert-Butyldimethylsilyloxy-29-des-(4-hydroxy-3-methylcyclohexyl)-29-(3-formylcyclopentyl)-FR 520**

[Formula I: $R_1$ = a group (d); $R_2$ = OtBDMS, single bond in 23,24 position; $R_3$ = Et; $R_4$ = OH, single bond in 10,11 position]

[Process variant protection]

A solution of 1.2 g **29-des-(4-hydroxy-3-methylcyclohexyl)-29-(3-formylcyclopentyl)-FR 520** - (compound of Example 12), 1.5 g tert-butyldimethylsilyl chloride and 0.8 g imidazole in 20 ml of dry dimethylformamide is stirred for 15 hours at room temperature and thereafter partitioned between 1 N hydrochloric acid solution and acetic acid ethyl ester. The organic phase is separated, washed with saturated aqueous sodium chloride solution, dried over sodium sulfate, filtered and concentrated under reduced pressure. The **title compound** is obtained from the residue as a colourless foamy resin following column chromatography over silicagel (eluant: n-hexane / acetic acid ethyl ester):

$^1$H-NMR: mixture of rotamers: 9.65 (d, J = 2 Hz, CHO); 5.39 (d, J = 9 Hz, H-29); 5.01 (d, J = 7.5 Hz, H-26); 4.81 (d, J = 10 Hz, H-20); 3.82 (dxd, J = 9/2 Hz, H-24).

The compounds of Examples 1 to 9 may be prepared in analogous manner according to process variant protection.

The compounds of the invention possess pharmacological activity. They are indicated for use as pharmaceuticals. In particular they possess antiinflammatory, and immunosuppressant and antiproliferative activity.

Antiinflammatory activity may e.g. be determined in the following test methods:

1. Oxazolone allergic contact dermatitis in the mouse in vivo upon topical application: the test method is as described in F.M. Dietrich and R. Hess, Int. Arch. Allergy 38 (1970) 246-259. The compounds elicit in this test an activity between about 15 % and about 68 % upon topical administration at a concentration of about 0.01 %.

2. DNFB allergy (swine): the test method is as described in e.g. EP 315978. Topical application of a 1.2 % formulation of the compounds repeated twice results in from about 36 % to about 40 % inhibition of the inflammatory reaction.

Immunosuppressant and antiproliferative activity may e.g. be determined in the following test methods:

1. Proliferative response of lymphocytes to allogen stimulation in the mixed lymphocyte reaction (MLR) in vitro: T. Meo, "The MLR in the Mouse", Immunological Methods, L. Lefkovits and B. Pernis, Eds., Academic Press, N.Y. (1979), 227-239.

   The compounds elicit in this test ($IC_{50}$) suppression of mixed lymphocytes at a dosage of from about < 0.0008 µg/ml to about 0.09 µg/ml.

2. Inhibition of the primary humoral immune response to sheep erythrocytes in vitro: the test method is as described in R.I. Mishell and R.W. Dutton, Science 153 (1966) 1004-1006; R.I. Mishell and R.W. Dutton, J. Exp. Med. 126 (1967) 423-442.

   The compounds are active in this test with an $IC_{50}$ of from about 0.0024 µg/ml to about 0.32 µg/ml.

3. Inhibition of proliferation of human keratinocytes: the test method is as described in e.g. EP 315978. The compounds are active in this test at concentrations of from about 1 µg/ml to about 10 µg/ml resulting in an inhibition of from about 30 % to about 90 %.

The compounds of the invention in free form and where such forms exist in pharmaceutically acceptable salt form are therefore indicated as antiinflammatory and as immunosuppressant and antiproliferative agents for use in the prevention and treatment of inflammatory conditions and of conditions requiring immunosuppression, such as

a) the prevention and treatment of
   - resistance in situations of organ or tissue transplantation, e.g. of heart, kidney, liver, bone marrow and skin,
   - graft-versus-host disease, such as following bone marrow grafts,
   - autoimmune diseases such as rheumatoid arthritis, systemic Lupus erythematosus, Hashimoto's thyroidis, multiple sclerosis, Myasthenia gravis, diabetes type I and uveitis,
   - cutaneous manifestations of immunologically-mediated illnesses;

b) the treatment of inflammatory and hyperproliferative skin diseases, such as psoriasis, atopical dermatitis, contact dermatitis and further eczematous dermatitises, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus and acne; and

c) Alopecia areata.

The compounds may be administered systemically or topically.

For these indications the appropriate dosage will, of course, vary depending upon, for example, the host, the mode of administration and the nature and severity of the condition being treated. However, in general, satisfactory results are indicated to be obtained systemically at daily dosages of from about 0.15 mg/kg to about 1.5 mg/kg animal body weight. An indicated daily dosage in the larger mammal is in the range from about 0.01 mg to about 100 mg, conveniently administered, for example, in divided doses up to four times a day or in retard form.

For topical use satisfactory results are obtained with local administration of a 1-3 % concentration of active substance several times daily, e.g. 2 to 5 times daily. Examples of indicated galenical forms are lotions, gels and creams.

The compounds of the invention may be administered by any conventional route, in particular enterally, e.g. orally, e.g. in the form of tablets or capsules, or topically, e.g. in the form of lotions, gels or creams.

Pharmaceutical compositions comprising a compound of the invention in association with at least one pharmaceutical acceptable carrier or diluent may be manufactured in conventional manner by mixing with a pharmaceutically acceptable carrier or diluent. Unit dosage forms contain, for example, from about 0.0025 mg to about 50 mg of active substance.

Topical administration is e.g. to the skin. A further form of topical administration is to the eye, for the treatment of immune-mediated conditions of the eye, such as: auto-immune diseases, e.g. uveitis, keratoplasty and chronic keratitis; allergic conditions, e.g. vernal conjunctivitis; inflammatory conditions and corneal transplants, by the topical administration to the eye surface of a compound of the invention in a pharmaceutically acceptable ophthalmic vehicle.

The ophthalmic vehicle is such that the compound is maintained in contact with the ocular surface for a sufficient time period to allow the compound to penetrate the corneal and internal regions of the eye, e.g. the anterior chamber, posterior chamber, vitreous body, aqueous humor, vitreous humor, cornea, iris/ciliary, lens, choroid/retina and sclera.

The pharmaceutically acceptable ophthalmic vehicle may be e.g. an ointment, vegetable oil, or an encapsulating material.

Whilst the antiinflammatory and immunosuppressant and antiproliferative activity is the main activity of the compounds of the invention they also possesses some degree of activity in increasing sensitivity to, or in increasing the efficacy of, chemotherapeutic drug therapy.

This activity may e.g. be determined according to the test methods described in EP 360760.

The compounds of the invention are therefore indicated for use in reversing chemotherapeutic drug resistance of varying types, e.g. acquired or innate, or in increasing sensitivity to administered drug therapy, e.g. as a means of reducing regular chemotherapeutic dosage levels, for example in the case of anti-neoplastic or cytostatic drug therapy, as a means of decreasing overall drug toxicity and, more especially, as a means of reversing or reducing resistance, including both inherent and acquired resistance, to chemotherapy.

Preferred in the above indications are the following compounds of the invention:

- 29-des-(4-hydroxy-3-methoxycyclohexyl)-29-(3-formylcyclopentyl)-FR 520 (compound of Example 12); and
- 33-epi-33-chloro-FR 520 (compound of Example 66a).

**Claims**

**Claims for the following Contracting States : AT, BE, CH, DE, DK, FR, GB, IT, LI, LU, NL, SE**

**1.** A compound of formula I

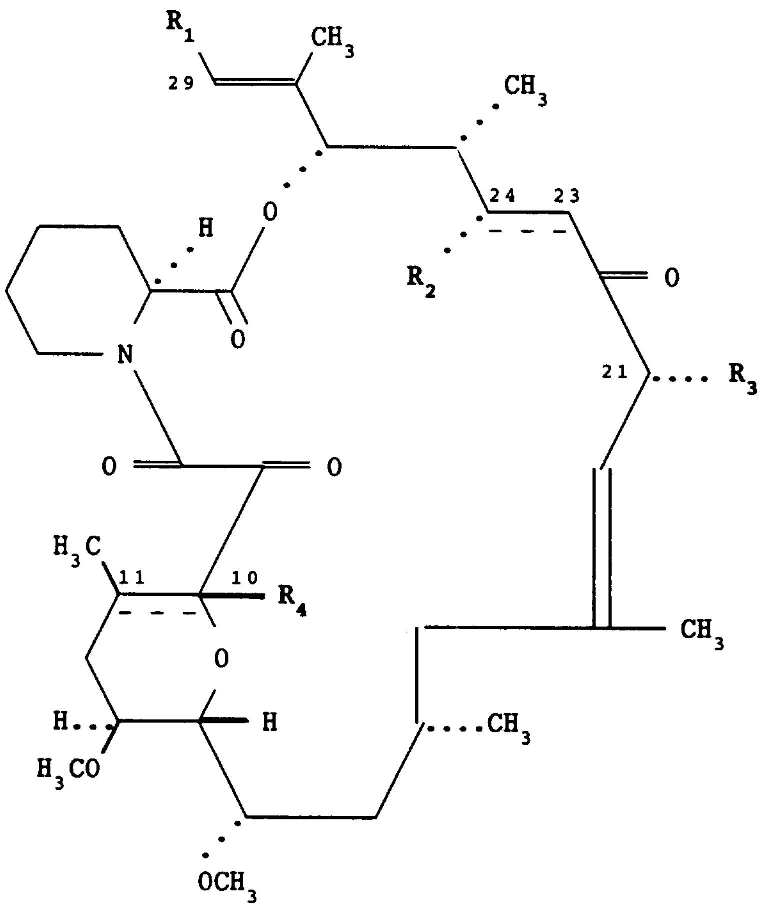

I

wherein
either

$R_1$ is a group (a) of formula

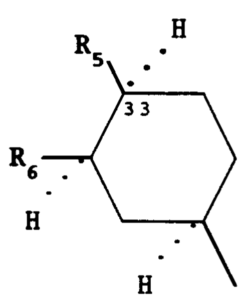

(a)

wherein

$R_5$ is chloro, bromo, iodo or azido and

$R_6$ is hydroxy or methoxy;

$R_2$ is oxo and there is a single bond in 23,24 position; optionally protected hydroxy and there is a single or a double bond in 23,24 position; or absent and there is a double bond in 23,24 position; and

$R_4$ is hydroxy and there is a single bond in 10,11 position; or absent and there is a double bond in 10,11 position;

or

$R_1$ is a group (b) or (d) of formula

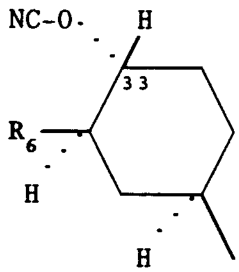
(b) and 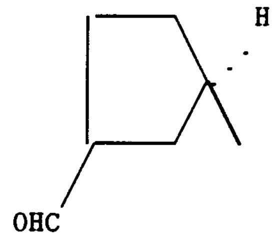 (d)

wherein

$R_6$ is as defined above;

$R_2$ is as defined above; and

$R_4$ is hydroxy and there is a single bond in 10,11 position; and

$R_3$ is methyl, ethyl, n-propyl or allyl;

in free form or, where such forms exist, in salt form.

2. A compound according to claim 1 which is a **compound $Ip_1$**,
i.e. a compound of formula I wherein

$R_1$ is a group (a) wherein $R_6$ is methoxy and

either

$R_5$ is chloro or bromo and

$R_4$ is hydroxy and there is a single bond in 10,11 position

or

$R_5$ is azido and

$R_4$ is hydroxy and there is a single bond in 10,11 position or absent and there is a double bond in 10,11 position;

$R_2$ is optionally protected hydroxy and there is a single or a double bond in 23,24 position; and

$R_3$ is as defined in claim 1;

in free form or, where such forms exist, in salt form.

3. A compound according to claim 1 which is a **compound $Ip_3$**,
i.e. a compound of formula I wherein

$R_1$ is a group (b) wherein $R_6$ is methoxy,

$R_2$ is optionally protected hydroxy and there is a single bond in 23,24 position; or absent and there is a double bond in 23,24 position;

$R_4$ is hydroxy and there is a single bond in 10,11 position; and

$R_3$ is as defined in claim 1;

in free form or, where such forms exist, in salt form.

4. A compound according to claim 1 which is a **compound $Ip_4$**,
i.e. a compound of formula I wherein

$R_1$ is a group (d),

$R_2$ is optionally protected hydroxy and there is a single bond in 23,24 position; or absent and there is a double bond in 23,24 position;

$R_4$ is hydroxy and there is a single bond in 10,11 position; and

$R_3$ is as defined in claim 1;

in free form or, where such forms exist, in salt form.

5. A compound of formula I according to claim 1 wherein R, is a group (d); $R_2$ is hydroxy and there is a single bond in 23,24 position; $R_3$ is ethyl; and $R_4$ is hydroxy and there is a single bond in 10,11 position; which is 29-des(4-hydroxy-3-methoxycyclohexyl)-29-(3-formylcyclopentyl)-FR 520 (compound of Example 12).

**6.** A compound of formula I according to claim 1 wherein $R_1$ is a group (a) wherein $R_5$ is chloro and $R_6$ is methoxy; $R_2$ is hydroxy and there is a single bond in 23,24 position; $R_3$ is ethyl; and $R_4$ is hydroxy and there is a single bond in 10,11 position; which is 33-epi-33-chloro-FR 520 (compound of Example 66a).

**7.** A process for the preparation of a compound according to claim 1 comprising

a) for the preparation of a compound of formula I wherein

$R_1$ is a group (a) as defined in claim 1,
$R_2$ and $R_3$ are as defined in claim 1 and
$R_4$ is hydroxy (i.e. a **compound Ia**),

replacing under simultaneous epimerization the hydroxy group by chlorine, bromine, iodine or azido in a corresponding compound having unprotected hydroxy in 33 position (i.e. a **compound IIa**, of formula IIa

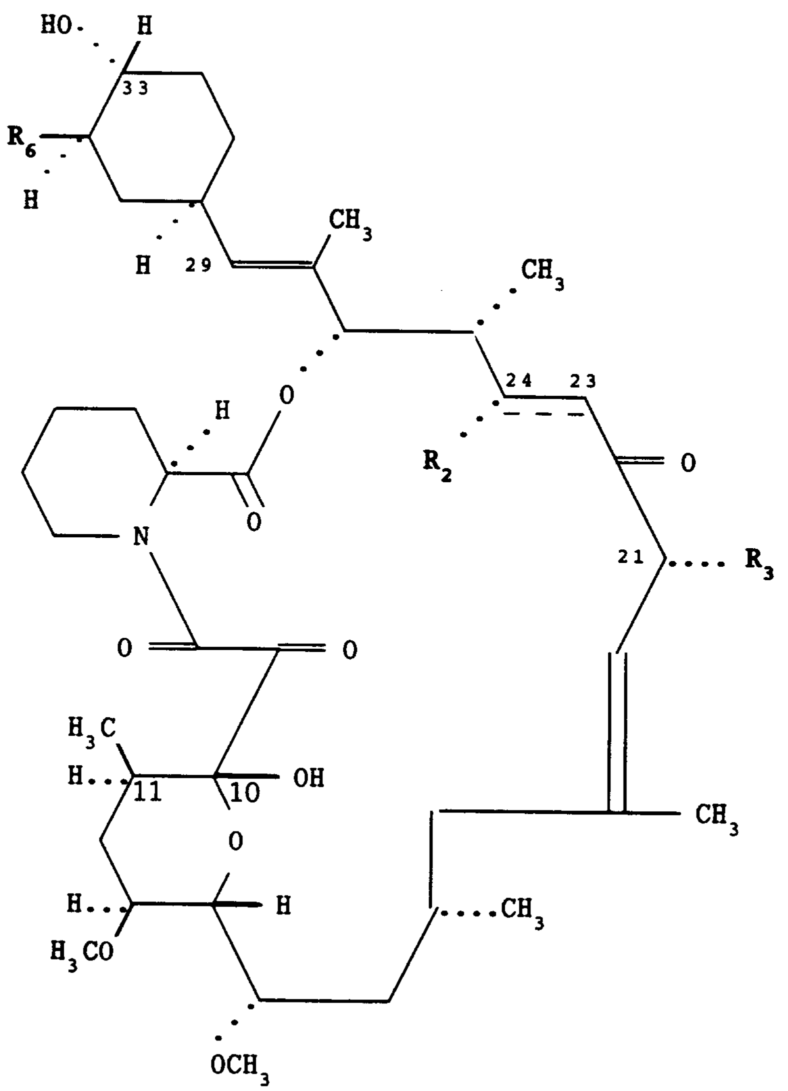

IIa

wherein $R_2$ and $R_3$ are as defined in claim 1 under formula I and $R_6$ is hydroxy or methoxy);

b) for the preparation of a compound of formula I wherein

$R_1$ is a group (b) as defined in claim 1,
$R_2$ and $R_3$ are as defined in claim 1 and
$R_4$ is hydroxy

(i.e. a **compound Ib**),

treating a corresponding compound IIa with cyanogen bromide in the presence of a base or

treating a corresponding compound IIa with thiophosgene, reacting the resultant product with an inorganic aside and allowing the resultant unstable intermediate having a group

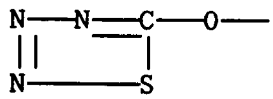

in 33 position (i.e. a **compound IIb**) to decompose to a corresponding compound Ib;

c) for the preparation of a compound of formula I wherein

$R_1$ is a group (d) as defined in claim 1,

$R_2$ and $R_3$ are as defined in claim 1 and

$R_4$ is hydroxy

(i.e. a **compound Ic**),

treating a corresponding compound Ib with an acid having a non-nucleophilic anion; and

- when a resultant compound of formula I has a protected hydroxy and/or a protected amino group, optionally splitting off the protecting group(s) to give a corresponding compound of formula I having one or more unprotected hydroxy and/or unprotected amino group(s) (i.e. a **compound Ij**),

  whereby when $R_1$ is a group (a), a water molecule may be simultaneously split off and a compound of formula I is obtained wherein

  $R_1$ is a group (a) as defined in claim 1,

  $R_2$ is unprotected hydroxy and there is a single or double bond in 23,24 position; and

  $R_4$ is absent and there is a double bond in 10,11 position

  (i.e. a **compound Ii**); or

- optionally protecting an unprotected hydroxy and/or unprotected amino group in a resultant compound of formula I as appropriate to give a corresponding compound of formula I having one or more protected hydroxy and/or protected amino groups(s) (i.e. a **compound Ik**);

and recovering the resultant compound of formula I in free form and, where such forms exist, in salt form.

**8.** A pharmaceutical composition comprising a compound according to any one of claims 1 to 6 in free form or, where such forms exist, in pharmaceutically acceptable salt form, together with a pharmaceutically acceptable carrier or diluent.

**9.** A compound according to any one of claims 1 to 6 in free form or, where such forms exist, in pharmaceutically acceptable salt form, for use as a pharmaceutical.

**10.** A compound according to claim 9 for use in the preparation of a pharmaceutical composition by mixing with a pharmaceutically acceptable carrier or diluent.

**11.** A process for the preparation of a pharmaceutical composition comprising mixing a compound according to any one of claims 1 to 6 in free form or, where such forms exist, in pharmaceutically acceptable salt form, with a pharmaceutically acceptable carrier or diluent.

**Claims for the following Contracting States : ES, GR**

**1.** A process for the preparation of a compound of formula I

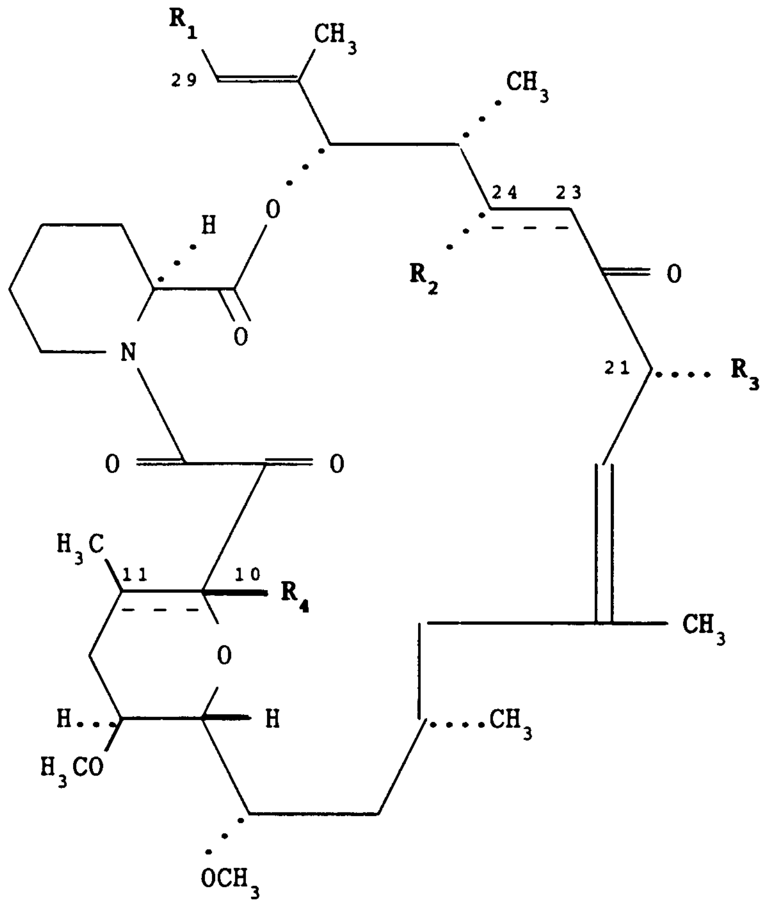

I

wherein
either

$R_1$ is a group (a) of formula

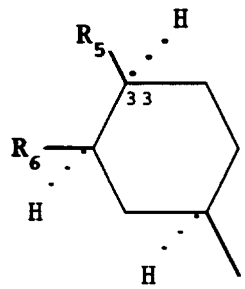

(a)

wherein

$R_5$ is chloro, bromo, iodo or azido and

$R_6$ is hydroxy or methoxy;

$R_2$ is oxo and there is a single bond in 23,24 position; optionally protected hydroxy and there is a single or a double bond in 23,24 position; or absent and there is a double bond in 23,24 position; and

$R_4$ is hydroxy and there is a single bond in 10,11 position; or absent and there is a double bond in 10,11 position;

or

$R_1$ is a group (b) or (d) of formula

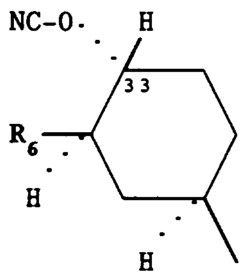 (b) and 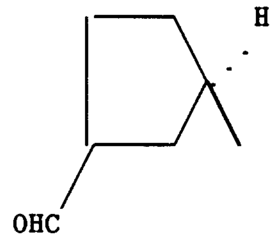 (d)

wherein

$R_6$ is as defined above;

$R_2$ is as defined above; and

$R_4$ is hydroxy and there is a single bond in 10,11 position; and

$R_3$ is methyl, ethyl, n-propyl or allyl;

in free form or, where such forms exist, in salt form,

comprising

a) for the preparation of a compound of formula I wherein

$R_1$ is a group (a) as defined in claim 1,

$R_2$ and $R_3$ are as defined in claim 1 and

$R_4$ is hydroxy (i.e. a **compound Ia**),

replacing under simultaneous epimerization the hydroxy group by chlorine, bromine, iodine or azido in a corresponding compound having unprotected hydroxy in 33 position (i.e. a **compound IIa**, of formula IIa

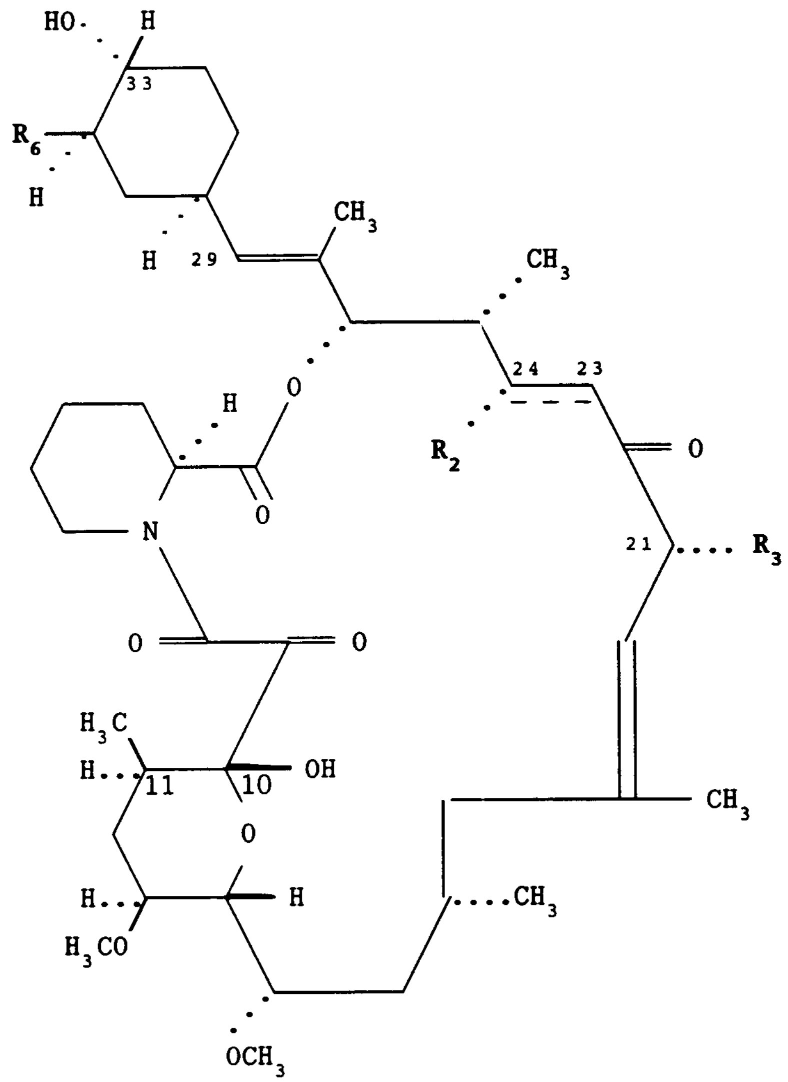

IIa

wherein $R_2$ and $R_3$ are as defined above under formula I and $R_6$ is hydroxy or methoxy);

b) for the preparation of a compound of formula I wherein

| | |
|---|---|
| $R_1$ | is a group (b) as defined in claim 1, |
| $R_2$ and $R_3$ | are as defined in claim 1 and |
| $R_4$ | is hydroxy |

(i.e. a **compound Ib**),

treating a corresponding compound IIa with cyanogen bromide in the presence of a base or

treating a corresponding compound IIa with thiophosgene, reacting the resultant product with an inorganic aside and allowing the resultant unstable intermediate having a group

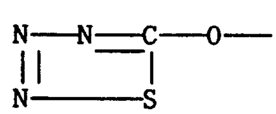

in 33 position (i.e. a **compound IIb**) to decompose to a corresponding compound Ib;

c) for the preparation of a compound of formula I wherein

| | |
|---|---|
| $R_1$ | is a group (d) as defined in claim 1, |
| $R_2$ and $R_3$ | are as defined in claim 1 and |
| $R_4$ | is hydroxy |

(i.e. a **compound Ic**),

treating a corresponding compound Ib with an acid having a non-nucleophilic anion; and

- when a resultant compound of formula I has a protected hydroxy and/or a protected amino group, optionally splitting off the protecting group(s) to give a corresponding compound of formula I having one or more unprotected hydroxy and/or unprotected amino group(s) (i.e. a **compound Ij**),

  whereby when $R_1$ is a group (a), a water molecule may be simultaneously split off and a compound of formula I is obtained wherein

  $R_1$ is a group (a) as defined in claim 1,

  $R_2$ is unprotected hydroxy and there is a single or double bond in 23,24 position; and

  $R_4$ is absent and there is a double bond in 10,11 position

  (i.e. a **compound Ii**); or
- optionally protecting an unprotected hydroxy and/or unprotected amino group in a resultant compound of formula I as appropriate to give a corresponding compound of formula I having one or more protected hydroxy and/or protected amino groups(s) (i.e. a **compound Ik**);

and recovering the resultant compound of formula I in free form and, where such forms exist, in salt form.

**2.** A process according to claim 1 for the preparation of the compound of formula I wherein $R_1$ is a group (d); $R_2$ is hydroxy and there is a single bond in 23,24 position; $R_3$ is ethyl; and $R_4$ is hydroxy and there is a single bond in 10,11 position; which is 29-des-(4-hydroxy-3-methoxycyclohexyl)-29-(3-formylcyclopentyl)-FR 520 (compound of Example 12).

**3.** A process according to claim 1 for the preparation of the compound of formula I wherein $R_1$ is a group (a) wherein $R_5$ is chloro and $R_6$ is methoxy; $R_2$ is hydroxy and there is a single bond in 23,24 position; $R_3$ is ethyl; and $R_4$ is hydroxy and there is a single bond in 10,11 position; which is 33-epi-33-chloro-FR 520 (compound of Example 66a).

**4.** A process for the preparation of a pharmaceutical composition comprising mixing a compound of formula I as defined in claim 1 in free form or, where such forms exist, in pharmaceutically acceptable salt form, with a pharmaceutically acceptable carrier or diluent.

**Patentansprüche**

**Patentansprüche für folgende Vertragsstaaten : AT, BE, CH, DE, DK, FR, GB, IT, LI, LU, NL, SE**

**1.** Verbindung der Formel I

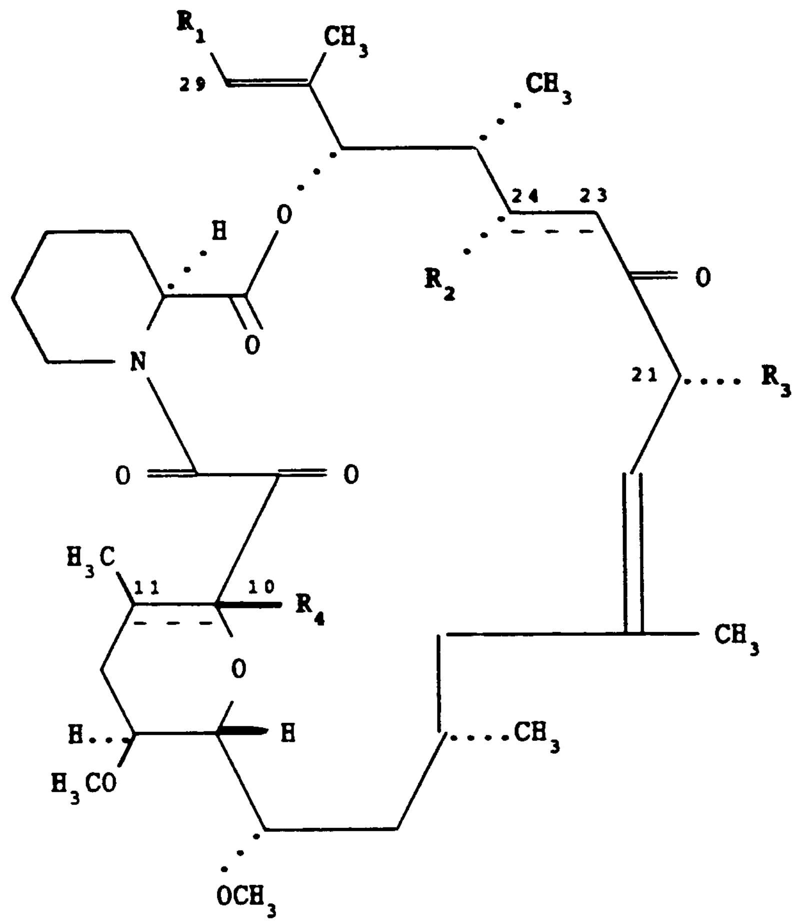

I

worin entweder

$R_1$ eine Gruppe (a) der Formel

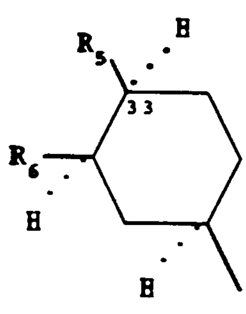

(a)

ist, worin

$R_5$ Chlor, Brom, Iod oder ein Azidorest ist und

$R_6$ ein Hydroxy- oder Methoxyrest ist;

$R_2$ ein Oxorest ist und in Position 23, 24 eine Einfachbindung ist; ein gegebenenfalls geschützter Hydroxyrest ist und in Position 23, 24 eine Einfach- oder Doppelbindung ist; oder nicht vorhanden ist und in Position 23, 24 eine Doppelbindung ist; und

$R_4$ ein Hydroxyrest ist und in Position 10, 11 eine Einfachbindung ist; oder nicht vorhanden ist und in Position 10, 11 eine Doppelbindung ist;

oder

$R_1$ eine Gruppe (b) oder (d) der Formel

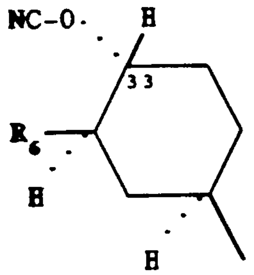

(b)

und

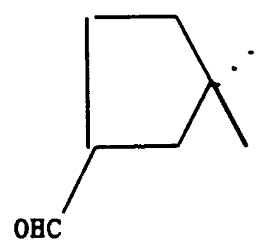

(d)

ist, worin $R_6$ wie oben definiert ist;

$R_2$ wie oben definiert ist; und

$R_4$ ein Hydroxyrest ist und eine Einfachbindung in Position 10, 11 ist; und

$R_3$ ein Methyl-, Ethyl-, n-Propyl- oder Allylrest ist;

in freier Form oder, wenn diese Formen existieren, in Salzform.

**2.** Verbindung nach Anspruch 1, die eine Verbindung $Ip_1$ ist, d.h. eine Verbindung der Formel I, worin

$R_1$ eine Gruppe (a) ist, worin $R_6$ ein Methoxyrest ist und entweder

$R_5$ Chlor oder Brom ist und

$R_4$ ein Hydroxyrest ist und in Position 10, 11 eine Einfachbindung ist oder

$R_5$ ein Azidorest ist und

$R_4$ ein Hydroxyrest ist und in Position 10, 11 eine Einfachbindung ist, oder nicht vorhanden ist und in Position 10, 11 eine Doppelbindung ist;

$R_2$ ein gegebenenfalls geschützter Hydroxyrest ist und in Position 23, 24 eine Einfach- oder Doppelbindung ist; und

$R_3$ wie in Anspruch 1 definiert ist;

in freier Form oder, wenn diese Formen existieren, in Salzform.

**3.** Verbindung nach Anspruch 1, die eine Verbindung $Ip_3$ ist, d.h. eine Verbindung der Formel I, worin

$R_1$ eine Gruppe (b) ist, worin $R_6$ ein Methoxyrest ist,

$R_2$ ein gegebenenfalls geschützter Hydroxyrest ist und in Position 23, 24 eine Einfachbindung ist; oder nicht vorhanden ist und in Position 23, 24 eine Doppelbindung ist;

$R_4$ ein Hydroxyrest ist und in Position 10, 11 eine Einfachbindung ist; und

$R_3$ wie in Anspruch 1 definiert ist;

in freier Form oder, wenn diese Formen existieren, in Salzform.

**4.** Verbindung nach Anspruch 1, die eine Verbindung $Ip_4$ ist, d.h. eine Verbindung der Formel I, worin

$R_1$ eine Gruppe (d) ist,

$R_2$ ein gegebenenfalls geschützter Hydroxyrest ist und in Position 23, 24 eine Einfachbindung ist; oder nicht vorhanden ist und in Position 23, 24 eine Doppelbindung ist;

$R_4$ ein Hydroxyrest ist und in Position 10, 11 eine Einfachbindung ist; und

$R_3$ wie in Anspruch 1 definiert ist;

in freier Form oder, wenn diese Formen existieren, in Salzform.

**5.** Verbindung der Formel I nach Anspruch 1, worin $R_1$ eine Gruppe (d) ist; $R_2$ ein Hydroxyrest ist und in Position 23, 24 eine Einfachbindung ist; $R_3$ ein Ethylrest ist; und $R_4$ ein Hydroxyrest ist und in Position 10, 11 eine Einfachbindung ist; die 29-des-(4-Hydroxy-3-methoxycyclohexyl)-29-(3-formylcyclo-pentyl)-FR 520 (Verbindung von Beispiel 12) ist.

**6.** Verbindung der Formel I nach Anspruch 1, worin $R_1$ eine Gruppe (a) ist, worin $R_5$ Chlor ist und $R_6$ ein Methoxyrest ist; $R_2$ ein Hydroxyrest ist und in Position 23, 24 eine Einfachbindung ist; $R_3$ ein Ethylrest ist; und $R_4$ ein Hydroxyrest ist und in Position 10, 11 eine Einfachbindung ist; die 33-epi-33-Chlor-FR 520 (Verbindung von Beispiel 66a) ist.

**7.** Verfahren zur Herstellung einer Verbindung nach Anspruch 1, umfassend,

a) zur Herstellung einer Verbindung der Formel I, worin

$R_1$ eine Gruppe (a), wie in Anspruch 1 definiert, ist,

$R_2$ und $R_3$ wie in Anspruch 1 definiert sind und

$R_4$ ein Hydroxyrest ist

(d.h. eine Verbindung Ia),

daß man unter gleichzeitiger Epimerisierung die Hydroxygruppe durch Chlor, Brom, Iod oder einen Azidorest in einer entsprechenden Verbindung mit einem ungeschützten Hydroxyrest in Position 33 ersetzt (d.h. eine Verbindung IIa der Formel IIa

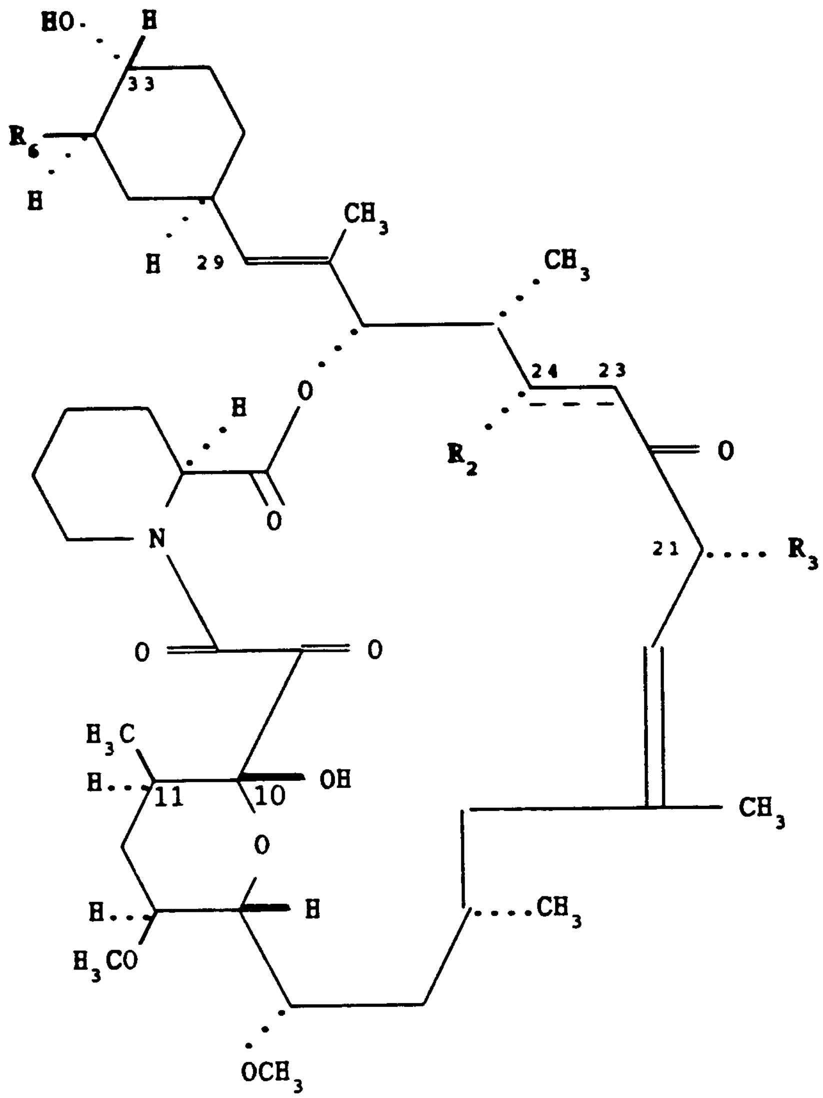

IIa

worin $R_2$ und $R_3$ wie in Anspruch 1 für Formel I definiert sind und $R_6$ ein Hydroxy- oder Methoxyrest ist);

b) zur Herstellung einer Verbindung der Formel I, worin

$R_1$ eine Gruppe (b), wie in Anspruch 1 definiert, ist,

$R_2$ und $R_3$ wie in Anspruch 1 definiert sind und

$R_4$ ein Hydroxyrest ist

(d.h. eine Verbindung Ib),

daß man eine entsprechende Verbindung IIa mit Bromcyan in Gegenwart einer Base behandelt oder eine entsprechende Verbindung IIa mit Thiophosgen versetzt, das entstehende Produkt mit einem anorganischen Azid umsetzt und das entstehende instabile Zwischenprodukt mit einer Gruppe

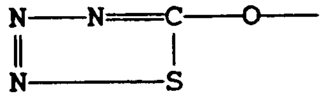

in Position 33 (d.h. eine Verbindung IIb) sich zu der entsprechenden Verbindung Ib zersetzen läßt;

c) zur Herstellung einer Verbindung der Formel I, worin

$R_1$ eine Gruppe (d), wie in Anspruch 1 definiert, ist,

$R_2$ und $R_3$ wie in Anspruch 1 definiert sind und

$R_4$ ein Hydroxyrest ist

(d.h. eine Verbindung Ic),

daß man eine entsprechende Verbindung Ib mit einer Säure mit einem nicht-nukleophilen Anion versetzt, und

- wenn eine entstehende Verbindung der Formel I eine geschützte Hydroxy- und/oder geschützte Aminogruppe hat, gegebenenfalls die Schutzgruppe(n) abspaltet, was eine entsprechende Verbindung der Formel I ergibt mit einer oder mehreren ungeschützten Hydroxy- und/oder ungeschützten Aminogruppen
  (d.h. eine Verbindung Ij),
  wobei dann, wenn $R_1$ eine Gruppe (a) ist, gleichzeitig ein Wassermolekül abgespalten werden kann und eine Verbindung der Formel I erhalten wird, worin
  $R_1$ eine Gruppe (a), wie in Anspruch 1 definiert, ist,
  $R_2$ ein ungeschützter Hydroxyrest ist und in Position 23, 24 eine Einfach- oder Doppelbindung ist; und
  $R_4$ nicht vorhanden ist und in Position 10, 11 eine Doppelbindung ist
  (d.h. eine Verbindung Ii); oder
- gegebenenfalls eine ungeschützte Hydroxy- und/oder ungeschützte Aminogruppe in einer entstehenden Verbindung der Formel I schützt, was eine entsprechende Verbindung der Formel I ergibt mit einer oder mehreren geschützten Hydroxy- und/oder geschützten Aminogruppen
  (d.h. eine Verbindung Ik),

und die entstehende Verbindung der Formel I in freier Form und, wenn diese Formen existieren, in Salzform gewinnt.

**8.** Pharmazeutische Zusammensetzung, umfassend eine Verbindung nach einem der Ansprüche 1 bis 6 in freier Form oder, wenn diese Formen existieren, in Form des pharmazeutisch annehmbaren Salzes zusammen mit einem pharmazeutisch annehmbaren Träger oder Verdünnungsmittel.

**9.** Verbindung nach einem der Ansprüche 1 bis 6 in freier Form oder, wenn diese Formen existieren, in Form des pharmazeutisch annehmbaren Salzes zur Vervendung als Pharmazeutikum.

**10.** Verbindung nach Anspruch 9 zur Verwendung zur Herstellung einer pharmazeutischen Zusammensetzung, indem sie mit einem pharmazeutisch annehmbaren Träger oder Verdünnungsmittel vermischt wird.

**11.** Verfahren zur Herstellung einer pharmazeutischen Zusammensetzung, umfassend, daß man eine Verbindung nach einem der Ansprüche 1 bis 6 in freier Form oder, wenn diese Formen existieren, in Form des pharmazeutisch annehmbaren Salzes mit einem pharmazeutisch annehmbaren Träger oder Verdünnungsmittel vermischt.

**Patentansprüche für folgende Vertragsstaaten : ES und GR**

1. Verfahren zur Herstellung einer Verbindung der Formel I

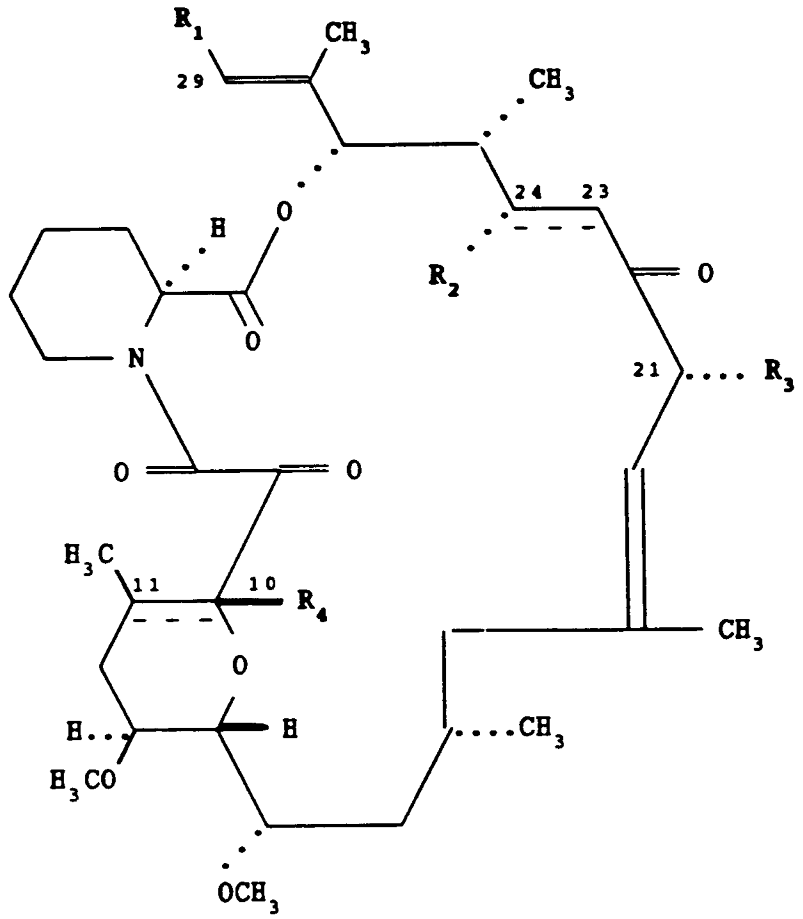

I

worin entweder

$R_1$ eine Gruppe (a) der Formel

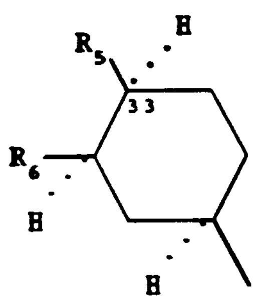

(a)

ist, worin

$R_5$ Chlor, Brom, Iod oder ein Azidorest ist und

$R_6$ ein Hydroxy- oder Methoxyrest ist;

$R_2$ ein Oxorest ist und in Position 23, 24 eine Einfachbindung ist; ein gegebenenfalls geschützter Hydroxyrest ist und in Position 23, 24 eine Einfach- oder Doppelbindung ist; oder nicht vorhanden ist und in Position 23, 24 eine Doppelbindung ist; und

$R_4$ ein Hydroxyrest ist und in Position 10, 11 eine Einfachbindung ist; oder nicht vorhanden ist und in Position 10, 11 eine Doppelbindung ist;

oder

$R_1$ eine Gruppe (b) oder (d) der Formel

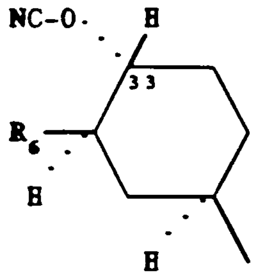 (b) und 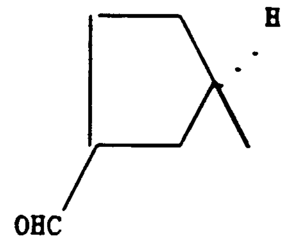 (d)

ist, worin $R_6$ wie oben definiert ist;

$R_2$ wie oben definiert ist; und

$R_4$ ein Hydroxyrest ist und eine Einfachbindung in Position 10, 11 ist; und

$R_3$ ein Methyl-, Ethyl-, n-Propyl- oder Allylrest ist;

in freier Form oder, wenn diese Formen existieren, in Salzform, umfassend, daß man

a) zur Herstellung einer Verbindung der Formel I, worin

$R_1$ eine Gruppe (a), wie in Anspruch 1 definiert, ist,

$R_2$ und $R_3$ wie in Anspruch 1 definiert sind und

$R_4$ ein Hydroxyrest ist

(d.h. eine Verbindung Ia),

daß man unter gleichzeitiger Epimerisierung die Hydroxygruppe durch Chlor, Brom, Iod oder einen Azidorest in einer entsprechenden Verbindung mit einem ungeschützten Hydroxyrest in Position 33 ersetzt (d.h. eine Verbindung IIa der Formel IIa

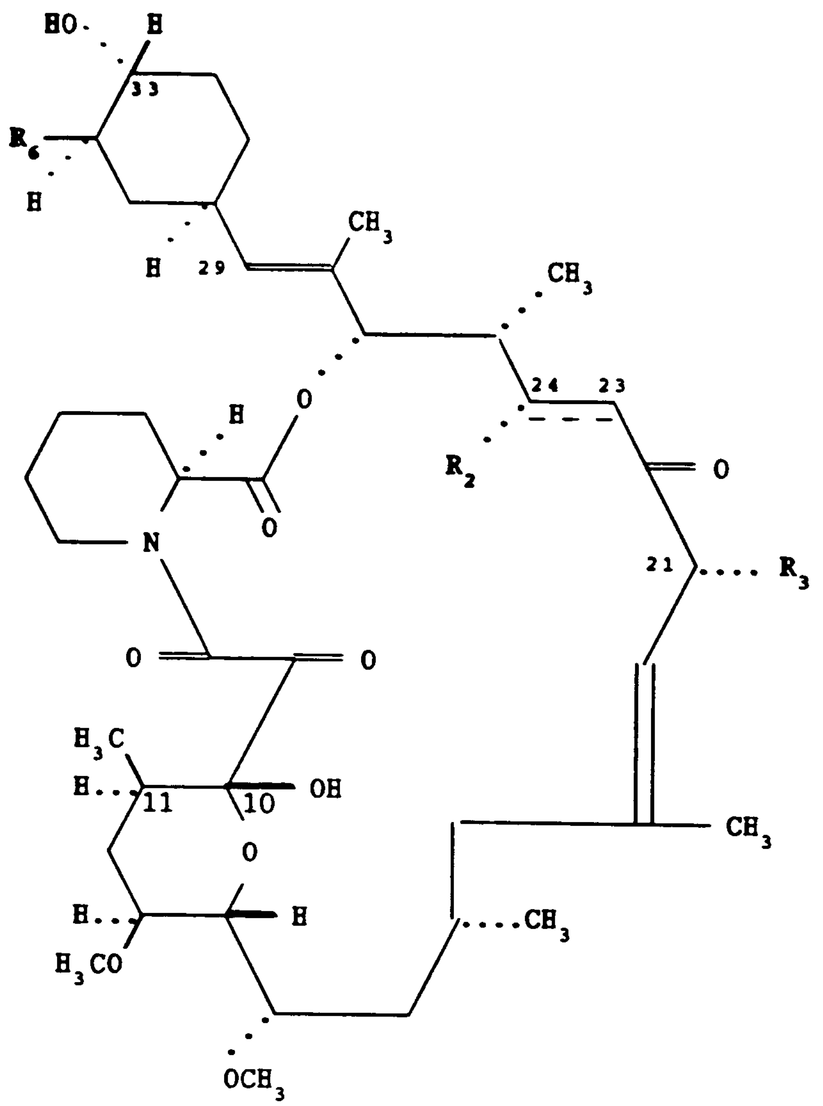

IIa

worin $R_2$ und $R_3$ wie oben für Formel I definiert sind und $R_6$ ein Hydroxy- oder Methoxyrest ist);

b) zur Herstellung einer Verbindung der Formel I, worin

$R_1$ eine Gruppe (b), wie in Anspruch 1 definiert, ist,

$R_2$ und $R_3$ wie in Anspruch 1 definiert sind und

$R_4$ ein Hydroxyrest ist

(d.h. eine Verbindung Ib),

daß man eine entsprechende Verbindung IIa mit Bromcyan in Gegenwart einer Base behandelt oder eine entsprechende Verbindung IIa mit Thiophosgen versetzt, das entstehende Produkt mit einem anorganischen Azid umsetzt und das entstehende instabile Zwischenprodukt mit einer Gruppe

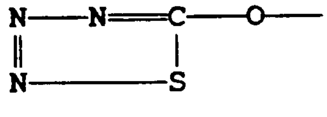

in Position 33 (d.h. eine Verbindung IIb) sich zu der entsprechenden Verbindung Ib zersetzen läßt;

c) zur Herstellung einer Verbindung der Formel I, worin

$R_1$ eine Gruppe (d), wie in Anspruch 1 definiert, ist,

$R_2$ und $R_3$ wie in Anspruch 1 definiert sind und

$R_4$ ein Hydroxyrest ist

(d.h. eine Verbindung Ic),

daß man eine entsprechende Verbindung Ib mit einer Säure mit einem nicht-nukleophilen Anion

versetzt, und

- wenn eine entstehende Verbindung der Formel I eine geschützte Hydroxy- und/oder geschützte Aminogruppe hat, gegebenenfalls die Schutzgruppe(n) abspaltet, was eine entsprechende Verbindung der Formel I ergibt mit einer oder mehreren ungeschützten Hydroxy- und/oder ungeschützten Aminogruppen
  (d.h. eine Verbindung Ij),
  wobei dann, wenn $R_1$ eine Gruppe (a) ist, gleichzeitig ein Wassermolekül abgespalten werden kann und eine Verbindung der Formel I erhalten wird, worin
  $R_1$ eine Gruppe (a), wie in Anspruch 1 definiert, ist,
  $R_2$ ein ungeschützter Hydroxyrest ist und in Position 23, 24 eine Einfach- oder Doppelbindung ist; und
  $R_4$ nicht vorhanden ist und in Position 10, 11 eine Doppelbindung ist
  (d.h. eine Verbindung Ii); oder
- gegebenenfalls eine ungeschützte Hydroxy- und/oder ungeschützte Aminogruppe in einer entstehenden Verbindung der Formel I schützt, was eine entsprechende Verbindung der Formel I ergibt mit einer oder mehreren geschützten Hydroxy- und/oder geschützten Aminogruppen
  (d.h. eine Verbindung Ik),

und die entstehende Verbindung der Formel I in freier Form und, wenn diese Formen existieren, in Salzform gewinnt.

**2.** Verfahren nach Anspruch 1 zur Herstellung der Verbindung der Formel I, worin $R_1$ eine Gruppe (d) ist; $R_2$ ein Hydroxyrest ist und in Position 23, 24 eine Einfachbindung ist; $R_3$ ein Ethylrest ist; und $R_4$ ein Hydroxyrest ist und in Position 10, 11 eine Einfachbindung ist; die 29-des-(4-Hydroxy-3-methoxycyclohexyl)-29-(3-formylcyclopentyl)-FR 520 (Verbindung von Beispiel 12) ist.

**3.** Verfahren nach Anspruch 1 zur Herstellung der Verbindung der Formel I, worin $R_1$ eine Gruppe (a) ist, worin $R_5$ Chlor ist und $R_6$ ein Methoxyrest ist; $R_2$ ein Hydroxyrest ist und in Position 23, 24 eine Einfachbindung ist; $R_3$ ein Ethylrest ist; und $R_4$ ein Hydroxyrest ist und in Position 10, 11 eine Einfachbindung ist; die 33-epi-33-Chlor-FR 520 (Verbindung von Beispiel 66a) ist.

**4.** Verfahren zur Herstellung einer pharmazeutischen Zusammensetzung, umfassend, daß man eine Verbindung der Formel I, wie in Anspruch 1 definiert, in freier Form oder, wenn diese Formen existieren, in Form des pharmazeutisch annehmbaren Salzes mit einem pharmazeutisch annehmbaren Träger oder Verdünnungsmittel vermischt.

**Revendications**

**Revendications pour les Etats contractants suivants : AT, BE, CH, DE, DK, FR, GB, IT, LI, LU, NL, SE**

**1.** Un composé de formule I

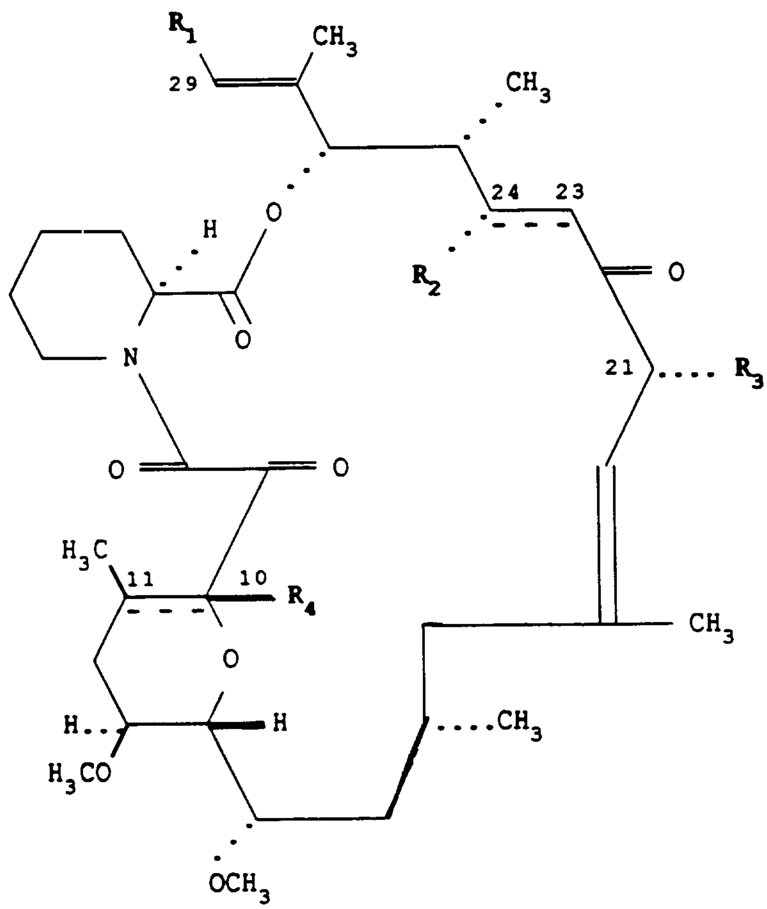

I

dans laquelle
soit

$R_1$ signifie un groupe (a) de formule

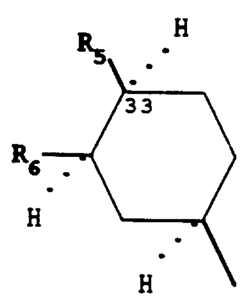

(a)

dans laquelle

$R_5$ signifie le chlore, le brome, l' iode ou un groupe azido, et

$R_6$ signifie un groupe hydroxy ou méthoxy,

$R_2$ signifie un groupe oxo et il existe une simple liaison en position 23,24; un groupe hydroxy éventuellement protégé et il existe une simple ou une double liaison en position 23,24; ou il est absent et il existe une double liaison en position 23,24, et

$R_4$ signifie un groupe hydroxy et il existe une simple liaison en position 10,11; ou il est absent et

il existe une double liaison en position 10,11; ou bien

$R_1$ signifie un groupe (b) ou (d) de formule

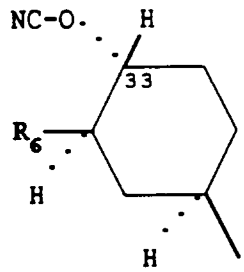

(b) et

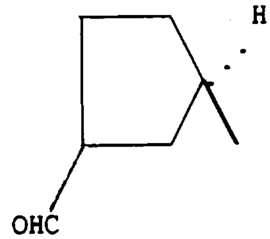

(d)

où $R_6$ est tel que défini plus haut,

$R_2$ est tel que défini plus haut, et

$R_4$ signifie un groupe hydroxy et il existe une simple liaison en position 10,11; et

$R_3$ signifie un groupe méthyle, éthyle, n-propyle ou allyle;

sous forme libre, ou, lorsqu'une telle forme existe, sous forme d'un sel.

**2.** Un composé selon la revendication 1, qui est un composé **Ip**$_1$, c'est-à-dire un composé de formule I où

$R_1$ signifie un groupe (a) où $R_6$ signifie un groupe méthoxy, et

soit

$R_5$ signifie le chlore ou le brome, et

$R_4$ signifie un groupe hydroxy et il existe une simple liaison en position 10,11, ou bien

$R_5$ signifie un groupe azido, et

$R_4$ signifie un groupe hydroxy et il existe une simple liaison en position 10,11 ou il est absent et il existe une double liaison en position 10,11,

$R_2$ signifie un groupe hydroxy éventuellement protégé et il existe une simple ou une double liaison en position 23,24, et

$R_3$ est tel que défini à la revendication 1,

sous forme libre, ou, lorsqu'une telle forme existe, sous forme d'un sel.

**3.** Un composé selon la revendication 1 qui est un Composé **Ip**$_3$, c'est-à-dire un composé de formule I où

$R_1$ signifie un groupe (b) où $R_6$ signifie un groupe méthoxy,

$R_2$ signifie un groupe hydroxy éventuellement protégé et il existe une simple liaison en position 23,24, ou il est absent et il existe une double liaison en position 23,24,

$R_4$ signifie un groupe hydroxy et il existe une simple liaison en position 10,11, et

$R_3$ est tel que défini à la revendication 1,

sous forme libre, ou, lorsqu'une telle forme existe, sous forme d'un sel.

**4.** Un composé selon la revendication 1 qui est un Composé **Ip**$_4$, c'est-à-dire un composé de formule I où

$R_1$ signifie un groupe (d),

$R_2$ signifie un groupe hydroxy éventuellement protégé et il existe une simple liaison en position 23,24 ou il est absent et il existe une double liaison en position 23,24,

$R_4$ signifie un groupe hydroxy et il existe une simple liaison en position 10,11 et

$R_3$ est tel que défini à la revendication 1,

sous forme libre, ou, lorsqu'une telle forme existe, sous forme d'un sel.

**5.** Un Composé de formule I selon la revendication 1, où $R_1$ signifie un groupe (d); $R_2$ signifie un groupe hydroxy et il existe une simple liaison en position 23,24; $R_3$ signifie un groupe éthyle; et $R_4$ signifie un groupe hydroxy et il existe une simple liaison en position 10,11; qui est le 29-dés-(4-hydroxy-3-méthoxycyclohexyle)-29-(3-formylcyclopentyle)-FR 520 (composé de l'exemple 12).

**6.** Un composé de formule I selon la revendication 1, où $R_1$ signifie un groupe (a) où $R_5$ signifie le chlore et $R_6$ signifie un groupe méthoxy; $R_2$ signifie un groupe hydroxy et il existe une simple liaison en position 23,24; $R_3$ signifie un groupe éthyle; et $R_4$ signifie un groupe hydroxy et il existe une simple liaison en position 10,11; qui est le 33-épi-33-chloro-FR 520 (composé de l'exemple 66a).

**7.** Un procédé de préparation d'un composé selon la revendication 1, comprenant

a) pour la préparation d'un composé de formule I où

| | |
|---|---|
| $R_1$ | signifie un groupe (a) tel que défini à la revendication 1, |
| $R_2$ et $R_3$ | sont tels que définis à la revendication 1, et |
| $R_4$ | signifie un groupe hydroxy (c'est-à-dire **un composé Ia**), |

on remplace sous épimérisation simultanée le groupe hydroxy par le chlore, le brome, l'iode ou un groupe azido dans un composé correspondant ayant un groupe hydroxy non protégé en position 33 (c'est-à-dire **un composé IIa,de formule IIa**

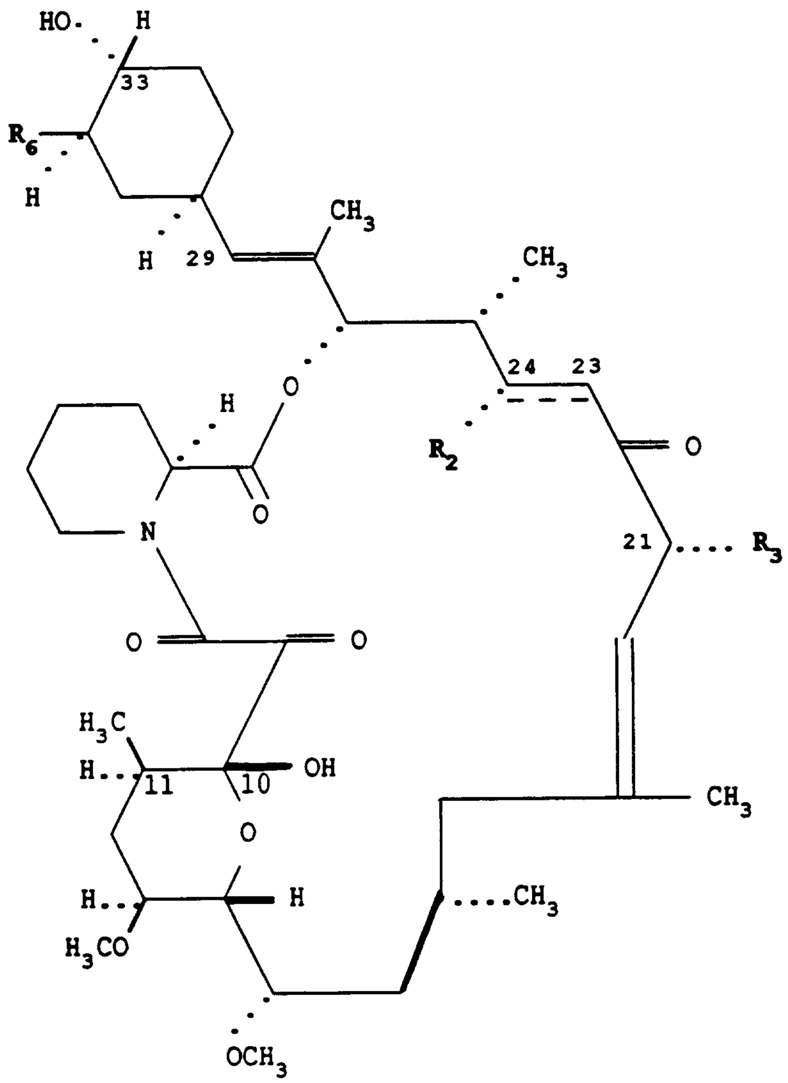

IIa

où $R_2$ et $R_3$ sont tels que définis à la revendication 1 sous la formule I, et $R_6$ signifie un groupe hydroxy ou méthoxy);

b) pour la préparation d'un composé de formule I où

| | |
|---|---|
| $R_1$ | signifie un groupe (b) tel que défini à la revendication 1, |
| $R_2$ et $R_3$ | sont tels que définis à la revendication 1, et |
| $R_4$ | signifie un groupe hydroxy |

(c'est-à-dire **un composé Ib**),

on traite un composé correspondant de formule IIa avec du bromure de cyanogène en présence d'une base ou bien

on traite un composé IIa correspondant avec du thiophosgène, on fait réagir le produit résultant avec un azidure minéral et on laisse se décomposer le produit intermédiaire instable résultant ayant un groupe

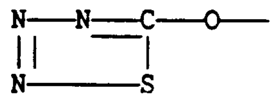

en position 33 (c'est-à-dire **un composé IIb**)
en un composé Ib correspondant;
c) pour la préparation d'un composé de formule I où
$R_1$ signifie un groupe (d) tel que défini à la revendication 1,
$R_2$ et $R_3$ sont tels que définis à la revendication 1, et
$R_4$ signifie un groupe hydroxy
(c'est-à-dire **un composé Ic**),
on traite un composé Ib correspondant avec un acide ayant un anion non nucléophile; et

- lorsqu'un composé résultant de formule I comporte un groupe hydroxy protégé et/ou un groupe amino protégé,
  on scinde éventuellement le ou les groupes protecteurs, ce qui donne un composé correspondant de formule I ayant un ou plusieurs groupes hydroxy non protégés et/ou un ou plusieurs groupes amino non protégés, (c'est-à-dire **un composé Ij**), et
  Lorsque $R_1$ signifie un groupe (a), une molécule d'eau peut être scindée simultanément et on obtient un composé de formule I où
  $R_1$ signifie un groupe (a) tel que défini à la revendication 1,
  $R_2$ signifie un groupe hydroxy non protégé et il existe une simple ou une double liaison en position 23,24, et
  $R_4$ est absent et il existe une double liaison en position 10,11,
  (c'est-à-dire **un composé Ii**); ou bien
- lorsque cela est approprié, on protège éventuellement un groupe hydroxy non protégé et/ou un groupe amino non protégé dans un composé résultant de formule I, ce qui donne un composé correspondant de formule I ayant un ou plusieurs groupes hydroxy protégés et/ou un ou plusieurs groupes amino protégés
  (c'est-à-dire **un composé Ik**);

et on récupère le composé résultant de formule I sous forme libre ou, lorsqu'une telle forme existe, sous forme d'un sel.

**8.** Une composition pharmaceutique comprenant un composé selon l'une quelconque des revendications 1 à 6 sous forme libre ou, lorsqu'une telle forme existe, sous forme d'un sel pharmaceutiquement acceptable, avec un véhicule ou diluant pharmaceutiquement acceptable.

**9.** Un composé selon l'une quelconque des revendications 1 à 6 sous forme libre ou, lorsqu'une telle forme existe, sous forme d'un sel pharmaceutiquement acceptable, pour l'utilisation comme médicament.

**10.** Un composé selon la revendication 9, pour une utilisation dans la préparation d'une composition pharmaceutique, par mélange avec un véhicule ou diluant pharmaceutiquement acceptable.

**11.** Un procédé de préparation d'une composition pharmaceutique comprenant le mélange d'un composé selon l'une quelconque des revendications 1 à 6, sous forme libre ou, lorsqu'une telle forme existe, sous forme d'un sel pharmaceutiquement acceptable, avec un véhicule ou diluant pharmaceutiquement acceptable.

**Revendications pour les Etats contractants suivants : ES, GR**

**1.** Un procédé de préparation d'un composé de formule I

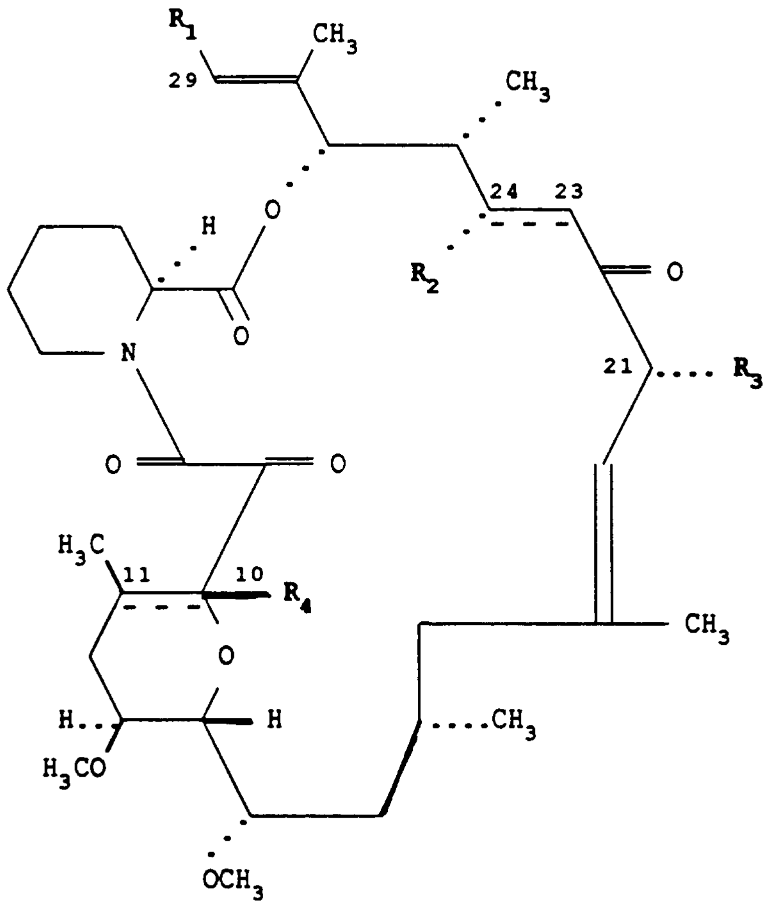

I

dans laquelle
soit

$R_1$ signifie un groupe (a) de formule

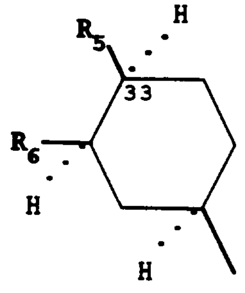

(a)

dans laquelle

$R_5$ signifie le chlore, le brome, l'iode ou un groupe azido, et

$R_6$ signifie un groupe hydroxy ou méthoxy,

$R_2$ signifie un groupe oxo et il existe une simple liaison en position 23,24; un groupe hydroxy éventuellement protégé et il existe une simple ou une double liaison en position 23,24; ou il est absent et il existe une double liaison en position 23,24; et

$R_4$ signifie un groupe hydroxy et il existe une simple liaison en position 10,11; ou il est absent et

il existe une double liaison en position 10,11; ou bien

$R_1$ signifie un groupe (b) ou (d) de formule

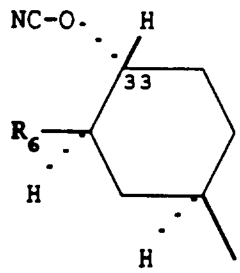 (b) et 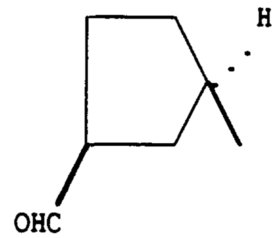 (d)

où

$R_6$ est tel que défini plus haut,

$R_2$ est tel que défini plus haut, et

$R_4$ signifie un groupe hydroxy et il existe une simple liaison en position 10,11, et

$R_3$ signifie un groupe méthyle, éthyle, n-propyle ou allyle,

sous forme libre, ou, lorsqu'une telle forme existe, sous forme d'un sel, comprenant

a) pour la préparation d'un composé de formule I où

$R_1$ signifie un groupe (a) tel que défini à la revendication 1,

$R_2$ et $R_3$ sont tels que définis à la revendication 1, et

$R_4$ signifie un groupe hydroxy (c'est-à-dire **un composé Ia**),

on remplace sous épimérisation simultanée le groupe hydroxy par le chlore, le brome, l'iode ou un groupe azido dans un composé correspondant ayant un groupe hydroxy non protégé en position 33 (c'est-à-dire **un composé IIa, de formule IIa**

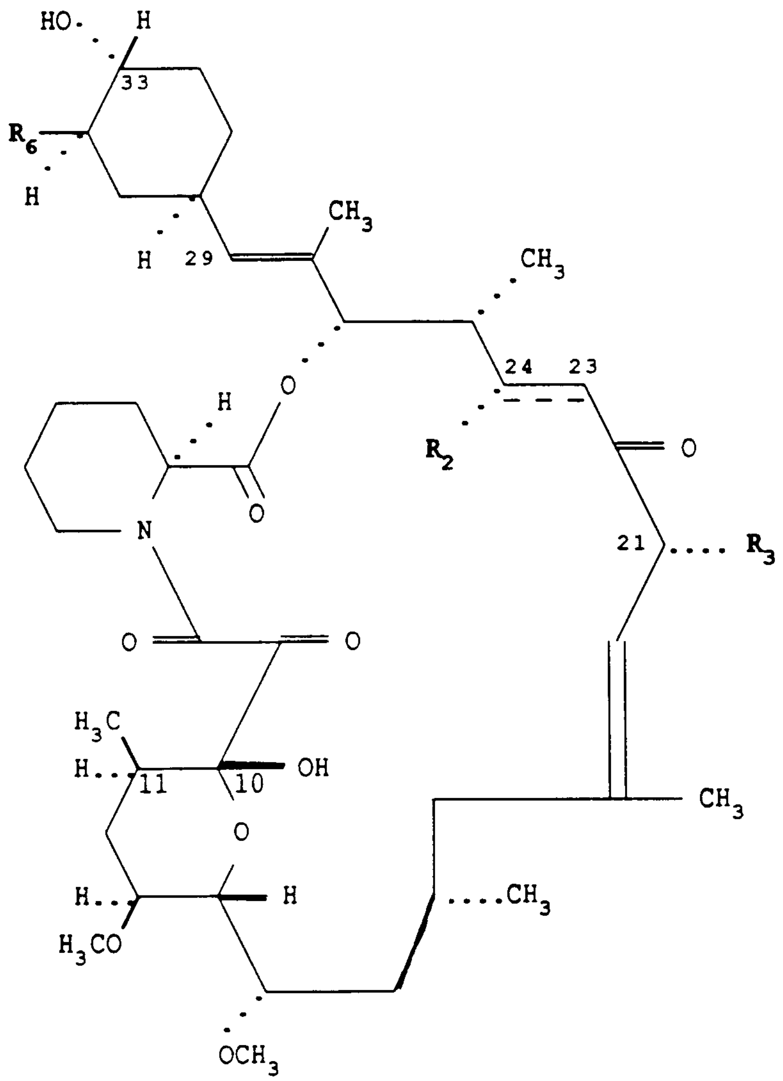

IIa

où $R_2$ et $R_3$ sont tels que définis plus haut sous la formule I, et $R_6$ signifie un groupe hydroxy ou méthoxy);

b) pour la préparation d'un composé de formule I où

$R_1$ signifie un groupe (b) tel que défini à la revendication 1,

$R_2$ et $R_3$ sont tels que définis à la revendication 1, et

$R_4$ signifie un groupe hydroxy

(c'est-à-dire **un composé Ib**),

on traite un composé correspondant de formule IIa avec du bromure de cyanogène en présence d'une base ou bien

on traite un composé IIa correspondant avec du thiophosgène, on fait réagir le produit résultant avec un azidure minéral et on laisse se décomposer le produit intermédiaire instable résultant ayant un groupe

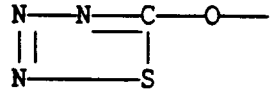

en position 33 (c'est-à-dire **un composé IIb**) en un composé Ib correspondant;

c) pour la préparation d'un composé de formule I où

$R_1$ signifie un groupe (d) tel que défini à la revendication 1,

$R_2$ et $R_3$ sont tels que définis à la revendication 1, et

$R_4$ signifie un groupe hydroxy
(c'est-à-dire **un composé Ic**),
on traite un composé Ib correspondant avec un acide ayant un anion non nucléophile, et

- lorsqu'un composé résultant de formule I comporte un groupe hydroxy protégé et/ou un groupe amino protégé,
  on scinde éventuellement le ou les groupes protecteurs, ce qui donne un composé correspondant de formule I ayant un ou plusieurs groupes hydroxy non protégés et/ou un ou plusieurs groupes amino non protégés, (c'est-à-dire **un composé Ij**), et
  Lorsque $R_1$ signifie un groupe (a), une molécule d'eau peut être scindée simultanément et on obtient un composé de formule I où
  $R_1$ signifie un groupe (a) tel que défini à la revendication 1,
  $R_2$ signifie un groupe hydroxy non protégé et il existe une simple ou une double liaison en position 23,24, et
  $R_4$ est absent et il existe une double liaison en position 10,11,
  (c'est-à-dire **un composé Ii**), ou bien
- lorsque cela est approprié, on protège éventuellement un groupe hydroxy non protégé et/ou un groupe amino non protégé dans un composé de formule I résultant, ce qui donne un composé correspondant de formule I ayant un ou plusieurs groupes hydroxy protégés et/ou un ou plusieurs groupes amino protégés

(c'est-à-dire **un composé Ik**),
et on récupère le composé résultant de formule I sous forme libre ou, lorsqu'une telle forme existe, sous forme d'un sel.

**2.** Un procédé selon la revendication 1 pour la préparation du composé de formule I, où $R_1$ signifie un groupe (d); $R_2$ signifie un groupe hydroxy et il existe une simple liaison en position 23,24; $R_3$ signifie un groupe éthyle; et $R_4$ signifie un groupe hydroxy et il existe une simple liaison en position 10,11; qui est le 29-dés-(4-hydroxy-3-méthoxycyclohexyle)-29-(3-formylcyclo-pentyle)-FR 520(composé de l'exemple 12).

**3.** Un procédé selon la revendication 1 pour la préparation du composé de formule I, où $R_1$ signifie un groupe (a) où $R_5$ signifie le chlore et $R_6$ signifie un groupe méthoxy; $R_2$ signifie un groupe hydroxy et il existe une simple liaison en position 23,24; $R_3$ signifie un groupe éthyle; et $R_4$ signifie un groupe hydroxy et il existe une simple liaison en position 10,11; qui est le 33-épi-33-chloro-FR 520 (composé de l'exemple 66a).

**4.** Un procédé de préparation d'une composition pharmaceutique comprenant le mélange d'un composé de formule I tel que défini à la revendication 1 sous forme libre ou, lorsqu'une telle forme existe, sous forme d'un sel pharmaceutiquement acceptable, avec un véhicule ou diluant pharmaceutiquement acceptable.